# EUROPÄISCHE PATENTSCHRIFT

(11) **EP 3 984 481 B1**
(45) Veröffentlichungstag und Bekanntmachung des Hinweises auf die Patenterteilung: **27.03.2024**
(21) Anmeldenummer: 21200970.8
(22) Anmeldetag: 05.10.2021
(51) Int. Cl.: A61B 18/12, A61B 18/00

(54) **TESTEINRICHTUNG**
TESTING DEVICE
DISPOSITIF DE TEST

(30) Priorität: 19.10.2020 DE 102020127424
(43) Veröffentlichungstag der Anmeldung: 20.04.2022
(73) Patentinhaber: Erbe Elektromedizin GmbH, 72072 Tübingen (DE)
(72) Erfinder: Fischer, Klaus, 72202 Nagold (DE); Neugebauer, Alexander, 72116 Moessingen (DE)
(74) Vertreter: Rüger Abel Patentanwälte PartGmbB

(56) Entgegenhaltungen:
- EP-B1- 1 737 402
- EP-B1- 2 659 846
- EP-B1- 2 815 695
- US-B1- 9 486 128

## Beschreibung

Die Erfindung betrifft das Gebiet der Analyse von Lichterscheinungen hervorgerufen durch elektrochirurgische Einwirkung auf Gewebe.

In dem in der Fachzeitschrift BIOMEDICAL OPTICS EXPRESS veröffentlichte Artikel der Autoren Spether et al., "Real-time tissue differentiation based on optical emission spectroscopy for guided electrosurgical tumor resection", wird eine vorklinische Studie beschrieben, in deren Rahmen zwischen einer Gewebeprobe, welche einem operativ entfernten Organ eines Patienten entnommen wurden, und einem Instrument eine Lichterscheinung erzeugt und anschließend untersucht wurde.

EP 2 815 695 B1 offenbart ein chirurgisches Instrument, das zur Gewebeerkennung von biologischem Gewebe eingerichtet ist. Licht, das während des Betriebs des elektrochirurgischen Instruments an einer Elektrode entsteht, wird durch eine Spektralanalyse bewertet und zuvor typisiertem Gewebe zugeordnet. Mittels einer Indikatoreinrichtung kann einem Operateur der Gewebetyp des aktuell behandelten Gewebes angezeigt werden.

In US 9 486 128 B1 ist ein elektrochirurgisches System beschrieben, bei dem ein Operationsbereich beleuchtet wird und ein Sensor vorhanden ist, der vom Operationsbereich reflektiertes Licht empfängt. Das empfangene Licht wird spektral analysiert, um einen Gewebetyp des im Operationsbereich vorhandenen Gewebes zu identifizieren.

Aus EP 2 659 846 A1 ist eine chirurgische Einrichtung mit einem Instrument zur Einwirkung auf biologisches Gewebe unter Erzeugung einer Lichterscheinung bekannt. Die Einrichtung weist eine Analyseeinrichtung zur Bestimmung von Lichtmerkmalen des durch die Einwirkung auf das elektrische Gewebe erzeugten Lichts und eine Diskriminatoreinrichtung zur Zuordnung von Lichtmerkmalen des bei der Einwirkung erzeugten Lichts zu Gewebemerkmalen des der Einwirkung unterliegenden biologischen Gewebes auf. EP 2 659 846 A1 schlägt vor, Referenzspektren oder charakteristische Lichtmerkmale von gesundem und krankem Gewebe, verschiedene Gewebetypen, usw. in einer Datenbank abzulegen. Die Datenbank kann statisch, d.h. vor Operationsbeginn mit entsprechenden Daten gefüllt sein. Es sei auch möglich, die Daten vor oder während der Operation zu gewinnen oder nachzujustieren. Beispielsweise kann vorgesehen sein, dass der Chirurg, solange er sicher in einem bestimmten Gewebe (z.B. in gesundem Gewebe) schneidet, wenigstens ein auftretendes Lichtmerkmal als Sollgröße abspeichert. Später erkannte Abweichungen von dieser Sollgröße können von der Diskriminatoreinrichtung erfasst werden, um ein Signal zu erzeugen. Die Einrichtung kann dieses Signal weiter verarbeiten und weitergeben. Allgemein kann gesagt werden, dass die Diskriminatoreinrichtung voreingestellte oder patientenindividuell gewonnene Referenzspektren oder einzelne Lichtmerkmale dieser Spektren mit einem gemessenen Spektrum oder einzelne Lichtmerkmalen desselben vergleicht und anhand von Abweichungen Signale erzeugt, die eine Änderung der Gewebeart oder Beschaffenheit anzeigen. Patientenindividuelle Lichtmerkmale, z.B. ein patientenindividuelles Referenzspektrum, können zu Beginn einer Operation oder auch ein- oder mehrfach während der Operation aufgenommen und gespeichert werden.

US 2009/0088772 A1 offenbart ein von einem Roboter getragenes chirurgisches Instrument, welches eine oder mehrere optische Fasern enthalten kann. Die optische Faser kann dafür verwendet werden, Photonen zu übertragen und zu empfangen und kann dazu eingerichtet sein, sowohl das Anregungslicht zu liefern als auch gestreute Photonen zu einer Analyseeinheit, z.B. einem Spektrophotometer, zu übertragen. Mit der optischen Faser kann optische Emissionsspektroskopie betrieben werden, um bestimmte chemische Spezies zu detektieren. Die Analyseeinheit kann mit einem Computer gekoppelt werden, um Parameter zur Kalibrierung der spektroskopischen Funktion des Instruments oder zum Testen der Diagnose einzuspeisen. Es kann eine Kalibrierung des Spektrophotometers unter Verwendung eines Standards, z.B. Standardsignalen von bekannten Lösungen oder der Gewebechemie, durchgeführt werden.

Aus EP 2 815 713 A1 ist ein Instrument zur elektrochirurgischen Einwirkung auf biologisches Gewebe bekannt. Dieses weist eine Elektrode sowie einen Lichtleiter auf, der mit einem von einem Fluidkörper gebildeten Lichteintrittsfenster verbunden ist. Der Lichtleiter ist mit einer Lichtanalyseeinrichtung verbunden, um das bei der HF-Chirurgie an der Elektrode entstehende Licht aufzunehmen und der Lichtanalyseeinrichtung zuzuleiten.

Lichtaufnahmeeinheiten wie beispielsweise optische Fasern können während des Betriebs verschmutzen, insbesondere durch Flüssigkeiten, Niederschlag von Rauch, Dampf und Partikel. Es tritt hinzu, dass ein System aus einer Analyseeinrichtung mit einer Lichtaufnahmeeinheit und einer Analyseeinheit sowie einem Instrument mit einer speziellen Elektrode und ein HF-Generator möglicherweise konkret erstmals bei einer bestimmten Operation zum Einsatz kommt.

Bei einem HF-Generator (Energiequelle für den HF-Funken) lässt die Norm beispielsweise eine Standardabweichung der HF-Ausgangsleistung von +/- 20% zu. Der spezifische elektrische Widerstand von biologischem Gewebe variiert stark, Fettgewebe hat 3,3×10⁷ Ohm mm²/m, Muskelgewebe 2,0×10⁶ Ohm mm²/m, und Blut dagegen nur 1,6×10⁶ Ohm mm²/m. Die Ausführung der verschiedenen Schneide- oder Koagulationsmode, wie z.B. bei einer Funkenregelung, haben Einfluss auf die Lichtemission des HF-Funkens am biologischen Gewebe. Die konkrete Lichtemission hängt also auch von den Einstellungen des HF-Generators bzw. des Instruments ab und zusätzlich von der Elektrodenform.

Die Ausführung des optischen Pfads des HF-OES-Systems von der Entstehung bis zur Analyseeinheit hat einen wesentlichen Einflussfaktor auf die Signalgüte des Lichtes. Die optischen Schnittstellen an den Steckkupplungen außerhalb und innerhalb der Analyseeinrichtung können die Transmission deutlich verschlechtern. Eine Verunreinigung der optischen Faser z.B. im Fertigungsprozess kann die Transmission verschlechtern oder sogar vollständig verhindern. Eine fehlerhafte mechanische Fixierung der optischen Fasern kann die Transmission deutlich verschlechtern. Fehler bei der Verbindung einer Analyseeinrichtung mit einem chirurgischen Instrument und/oder bei der Ausrichtung zueinander können dazu führen, dass kein brauchbares Lichtsignal von der Analyseeinrichtung aufgenommen werden kann. Auch im klinischen Einsatz eines HF-OES-Systems (Hochfrequenz-Optische-Emissionsspektroskopie-System) kann sich die Signalgüte eines HF-Funkens durch z.B. Verschmutzung der optischen Faser durch Blut deutlich verändern und somit eine Gewebedifferenzierung verfälschen oder unmöglich machen.

Aufgrund des Energieeintrags kann es bei der optischen Emissionsspektroskopie unter Anwendung der HF-Funken zur Gewebedifferenzierung zu einer großen Varianz in den Intensitäten kommen, insbesondere zu unterschiedlich starken HF-Funken. Die hohe Temperatur zu Anfang einer Funkenentladung führt zu Druckerhöhung, die wiederum den Querschnitt des Ladungskanals explosionsartig vergrößert. Dadurch sinkt die Spitzentemperatur. Temperatur und Druck um Plasma ist bei der Funkenanregung nicht konstant. Die Impedanz vom Plasma zwischen Elektrode und Gewebe variiert. Um die HF-OES-Spektren miteinander vergleichen zu können, müssen die HF-OES-Spektren jeweils ein Gütekriterium erfüllen, um die Gewebespektren in auswertbare und nicht auswertbare Spektren zu sortieren. Es werden keine Absolutwerte der Signalgüte, sondern nur Verhältnisse der einzelnen Spektrallinien zueinander gebildet. Der HF-Funke entsteht nicht nur zwischen Elektrodenspitze und Gewebe, sondern durch Verschmutzung können auch Funken seitlich außerhalb des Betrachtungsfensters der optischen Faser entstehen, so dass z.B. nur ein geringerer Teil der Lichtemission als ohne Verschmutzung in die Glasfaser eingekoppelt wird.

Tests oder Kalibrierungen von einzelnen Komponenten eines Systems sind aufgrund der Vielzahl von Kombinationsmöglichkeiten von Elektrode, Lichtleiter, Analyseeinheit, HF-Generator, Einstellparametern usw. möglicherweise nur beschränkt aussagekräftig.

Es ist Aufgabe der vorliegenden Erfindung, ein verbessertes Konzept für ein System mit einer Analyseeinrichtung zur Analyse von Lichterscheinungen, hervorgerufen durch ein HF-chirurgisches Instrument, anzugeben.

Diese Aufgabe wird mit einer Testeinrichtung zum Kalibrieren einer Analyseeinrichtung zur Analyse von Lichterscheinungen - hervorgerufen durch ein HF-chirurgisches Instrument - nach Anspruch 1 gelöst. Zu der Testeinrichtung gehört wenigstens ein Testobjekt. Das Testobjekt ist zum Erzeugen einer Plasmatestlichterscheinung eingerichtet, deren Licht durch eine Lichtaufnahmeeinheit aufnehmbar ist. Die Testeinrichtung ist zum Kalibrieren wenigstens von Teilen der Analyseeinrichtung oder der gesamten Analyseeinrichtung eingerichtet. Die Analyseeinrichtung kann eine Lichtaufnahmeeinheit, beispielsweise eine optische Faser, aufweisen, und eine Analyseeinheit, wobei die Lichtaufnahmeeinheit mit der Analyseeinheit verbunden ist. Die Analyseeinrichtung dient insbesondere zur Gewebeunterscheidung (Gewebedifferenzierung), beispielsweise zwischen gesundem und malignem Gewebe.

Das Testobjekt der Testeinrichtung stellt eine körperfremde (bezogen auf jeden menschlichen oder tierischen Körper eines Lebewesens, insbesondere den Körper des Patienten, welcher mit dem Instrument behandelt werden soll) Testoberfläche bereit, so dass das Testverfahren mittels der Testeinrichtung nicht am menschlichen oder tierischen Lebewesen durchgeführt wird.

Das Testobjekt besteht vorzugsweise aus nichtbiologischem Material. Insbesondere weist das Testobjekt vorzugsweise kein menschliches oder tierisches Gewebe auf oder es wird als Testobjekt oder Teil des Testobjekts vorzugsweise kein menschliches oder tierisches Gewebe bereitgestellt. Dies schließt natürlich nicht aus, dass das Testobjekt beim Testen während einer Operation mit Geweberesten oder dergleichen verunreinigt werden kann.

Unter einer Plasmatestlichterscheinung (kurz Testlichterscheinung) wie sie mittels des Testobjekts erzeugt wird, wird beispielsweise ein Funken oder ein stehendes Plasma verstanden. Die Lichterscheinung kann elektromagnetische Wellen im infraroten, sichtbaren und/oder ultravioletten Teil des elektromagnetischen Spektrums enthalten. Die Testeinrichtung kann zum Aufnehmen und Analysieren von infraroten, sichtbaren und/oder ultravioletten Beiträgen zum Spektrum der Testlichterscheinung eingerichtet sein. Die Lichtaufnahmeeinheit, welche die Plasmatestlichterscheinung aufnimmt, kann eine Testlichtaufnahmeeinheit sein, welche mit der Analyseeinheit der Analyseeinrichtung verbunden ist oder testweise verbunden werden kann. Bevorzugt aber ist die Lichtaufnahmeeinheit die Lichtaufnahmeeinheit der Analyseeinrichtung. Denn bevorzugt wird mittels der Testeinrichtung die Analyseeinrichtung in Kombination mit dem Instrument und besonders bevorzugt dem HF-Chirurgiegerät zur Speisung des Instruments mit HF-Energie getestet, welche auch bei der konkreten Operation verwendet werden, zu deren Vorbereitung oder während derer die Analyseeinrichtung kalibriert wird.

Ein medizinisch geschultes Mitglied des Operationsteams, z.B. ein Operationsassistent oder ein Chirurg kann sich mittels der Testeinrichtung bevorzugt vor oder während der Operation von der grundsätzlichen Funktionsfähigkeit der Analyseeinrichtung überzeugen, indem er eine Testlichterscheinung mittels des Testobjekts erzeugt und das Licht durch eine Lichtaufnahmeeinheit aufnimmt. Die Testeinrichtung ist bevorzugt dazu eingerichtet, ein Testergebnis des Funktionstests, beispielsweise "Test erfolgreich ", d.h. die Analyseeinrichtung ist funktionsfähig, und/oder "Test nicht erfolgreich", d.h. die Analyseeinrichtung ist nicht funktionsfähig, durch ein entsprechendes Signal oder das Ausbleiben eines Signals insbesondere eines optischen, akustischen oder haptisch wahrnehmbaren Signals an das Operationspersonal auszugeben. Damit können beispielsweise Fälle aufgedeckt werden, in denen eine optische Faser der Analyseeinrichtung verschmutzt oder defekt ist oder in denen die Analyseeinrichtung nicht korrekt zusammengesetzt ist.

Weitere beispielhafte vorteilhafte Merkmale und Ausführungsformen der erfindungsgemäßen Testeinrichtung, des erfindungsgemäßen Verfahrens und erfindungsgemäßer Systeme ergeben sich aus nachfolgender Beschreibung:

Das Testobjekt kann eine Testoberfläche zur Erzeugung der Testlichterscheinung zwischen einer Elektrode, bevorzugt einer Elektrode des Instruments, und der Testoberfläche aufweisen. Die Testoberfläche kann von einer Gegenelektrode zu der Elektrode gebildet werden. Die Testoberfläche kann elektrisch leitend mit dem HF-chirurgischen Gerät und/oder einer Neutralelektrode, wenn es sich um ein monopolares Instrument handelt, elektrisch leitend verbunden sein.

Die Testeinrichtung ist bevorzugt für den klinischen Einsatz eingerichtet. Die Testeinrichtung ist bevorzugt zur Verwendung während der intraoperativen Phase der Operation mit dem chirurgischen Instrument eingerichtet. Besonders bevorzugt ist das Testobjekt in einem sterilen Operationsbereich anordenbar und/oder angeordnet. Damit wird der Test, insbesondere die Kalibration, der Analyseeinrichtung mittels der Testeinrichtung unmittelbar vor oder während der Operation besonders einfach möglich.

Bevorzugt weist die Testeinrichtung eine Auswerteeinheit auf, welche dazu eingerichtet ist, anhand von Lichtmerkmalen (einem Lichtmerkmal oder mehrere Lichtmerkmalen) des aufgenommenen Lichtes einer Testlichterscheinung, welche mittels des Testobjekts erzeugt ist, zu bewerten, ob die Analyseeinrichtung funktionsfähig ist. Die Auswerteeinheit kann beispielsweise Teil der Analyseeinrichtung sein.

Lichtmerkmale zur Bewertung, ob die Analyseeinrichtung funktionsfähig ist, können beispielsweise sein: Lichtleistung nicht spektral aufgelöst bzw. integriert über alle aufgenommenen Wellenlängen, Lichtleistung bei einer Wellenlänge oder mehreren voneinander unterscheidbaren (Abstand größer als die Wellenlängenauflösung der Testeinrichtung) Wellenlängen, Intensitäten einzelner Spektrallinien, Intensitäten von Spektralliniengruppen, zeitlicher Verlauf der Testlichterscheinung, Polarisation des Lichts der Testlichterscheinung, typische Spektren, Ausschnitte von Spektren, aus Spektren gewonnene Signaturen, und/oder Signaturen, die aus dem Spektrum des Lichts der Testlichterscheinung, Ausschnitten des Spektrums und/oder Spektrallinien gewonnen sind. Signaturen können als Rechengrößen aus Algorithmen, Formeln, Zuordnungstabellen oder sonstigen Verarbeitungsvorschriften gewonnen werden. Insbesondere können Signaturen: Intensitätsverhältnisse ausgewählter Spektrallinien, Verhältnisse, Summen und/oder Differenzen zwischen verschiedenen Termen sein, wobei die Terme ihrerseits wiederum: Summen, Differenzen, Produkte, Integrale über Teilspektren und/oder andere Terme von Intensitäten verschiedener Spektrallinien oder Teilspektren oder davon abgeleitete Lichtmerkmale oder Signaturen sind.

Bewertungskriterium kann beispielsweise Signalzu- Rauschen-Verhältnis oder Signal-zu-Untergrund-Verhältnis bei einer Wellenlänge, Gruppen von Wellenlängen, Abschnitten des Spektrums, des gesamten Spektrums, Verhältnisse von Intensitäten bei unterschiedlichen Wellenlängen und/oder Wellenlängengruppen und/oder Abschnitten des Spektrums sein. In einem einfachen Fall kann beispielsweise aus der mittels des Testeinrichtung ermittelten Intensität des Lichts der Testlichterscheinung durch Vergleich mit einem Schwellwert ermittelt werden, ob genügend Licht durch die Lichtaufnahmeeinheit aufgenommen werden kann, so dass die Analyseeinrichtung funktionsfähig ist.

Alternativ oder zusätzlich kann beispielsweise eine Signatur aus Intensitätsverhältnissen ausgewählter Spektrallinien des Lichts der Testlichterscheinung berechnet werden und/oder die Unsicherheit bei der Berechnung der Signatur aufgrund beispielsweise von Rauschen oder Untergrund ermittelt werden. Beispielsweise kann jede Signatur aus Intensitätsverhältnissen ausgewählter für in den Plasmazustand übergegangener Teile des Materials der Testoberfläche und/oder der Elektrode und/oder der Atmosphäre zwischen der Elektrode und der Testoberfläche kennzeichnende Spektrallinien berechnet sein. Ist die Unsicherheit, beispielsweise das Verhältnis aus Signatur zu Unsicherheit, größer als ein Schwellwert, kann beispielsweise von einem positiven Test der Analyseeinrichtung ausgegangen werden, wobei die Auswerteeinheit ein entsprechendes Bewertungssignal generiert. Bei negativem Testausgang kann die Auswerteeinheit ein entsprechendes anderes Bewertungssignal oder kein Bewertungssignal erzeugen oder alternativ bleibt die Bewertungssignalausgabe bei einem positiven Testergebnis aus. Aufgrund des Bewertungssignals oder des anderen Bewertungssignals kann an den Anwender des chirurgischen Instruments und/oder der Analyseeinrichtung ein insbesondere akustisch, optisch oder haptisch wahrnehmbares Signal ausgegeben werden und/oder das Bewertungssignal kann zur Steuerung des Analyseeinrichtungstestsystems, der Analyseeinrichtung, des HF-chirurgischen Instruments und/oder des HF-chirurgischen Geräts herangezogen werden. Aufgrund des Bewertungssignals können beispielsweise automatisch vom System Parameter oder Parameterbereiche der Testeinrichtung, der Analyseeinrichtung, des HF-chirurgischen Instruments und/oder des HF-chirurgischen Geräts vorgegeben werden. Dies bedeutet, dass das HF-Chirurgiesystem mit HF-chirurgischem Gerät, HF-chirurgischem Instrument, Testeinrichtung, Analyseeinrichtung und/oder gegebenenfalls weiteren Einrichtungen auf Grund des Tests von der Testeinrichtung automatisch justiert werden kann (mit oder ohne Freigabe der automatischen Justierung durch den Benutzer), indem wenigstens eine Einstellung wenigstens einer Einrichtung des HF-Chirurgiesystems auf Grund des Tests gewählt oder verändert wird. Die Testeinrichtung kann alternativ oder zusätzlich auf Grund des Tests eine Empfehlung für wenigstens eine Einstellung wenigstens einer Einrichtung des HF-Chirurgiesystems an den Benutzer ausgeben, welcher dann entscheiden kann, das HF-Chirurgiesystem entsprechend der Empfehlung zu justieren.

Aufgrund des Bewertungssignals kann beispielsweise verhindert werden, dass die Analyseeinrichtung ein Signal ausgibt und/oder es kann beispielsweise eine Angabe zu einer Genauigkeit der Analyse der Analyseeinrichtung abgeleitet und an den Anwender ausgegeben werden. Bewertungsergebnis kann also beispielsweise sein, ob die Analyseeinrichtung überhaupt arbeitet, mit ausreichender Genauigkeit arbeitet und/oder mit welcher Genauigkeit die Analyseeinrichtung in der Lage ist, zu arbeiten.

Die Testeinrichtung ist bevorzugt dazu eingerichtet, anhand der Testlichterscheinung eine Empfindlichkeit der Analyseeinrichtung bei wenigstens einer Wellenlänge, einer Gruppe von Wellenlängen, in einem Teilspektrum oder dem Gesamtspektrum und/oder eine Transmissivität der Analyseeinrichtung bei wenigstens einer Wellenlänge, einer Gruppe von Wellenlängen, einem Teilspektrum oder des Gesamtspektrums zu ermitteln und bevorzugt zu bewerten. Die Empfindlichkeit ist die Größe des elektrischen Signals in der Analyseeinrichtung, hervorgerufen durch das aufgenommene Licht der Testlichterscheinung im Verhältnis zur Intensität des Lichtes der Testlichterscheinung. Die Transmissivität ist das Verhältnis der Intensität am Lichtausgang der Lichtaufnahmeeinheit (beispielsweise einer optischen Faser) zur Intensität am Lichteingang der Lichtaufnahmeeinheit. Die Transmissivität kann beispielsweise durch Verschmutzung des Eingangs, Beschädigung, beispielsweise Bruch zwischen einschließlich dem Lichteingang und dem Lichtausgang (dort, wo das Lichtsignal in ein elektrisches Signal umgesetzt wird) und/oder beispielsweise bei Kopplungsstücken zwischen dem Lichteingang und dem Ausgang beeinträchtigt sein. In einer Datenbank können Vergleichsempfindlichkeiten und/oder Transmissivitäten hinterlegt sein, mit denen die ermittelte Empfindlichkeit und/oder ermittelte Transmissivität von der Testeinrichtung verglichen werden kann. Da bei der Transmissivitätsbestimmung Untersuchungsobjekt die Lichtaufnahmeeinheit der Analyseeinrichtung ist, kann bei der Ermittlung der Transmissivität die Analyseeinheit der Analyseeinrichtung verwendet werden oder eine andere Analyseeinheit testweise mit der Lichtaufnahmeeinheit verbunden sein.

Bevorzugt weist das Testobjekt wenigstens einen oder wenigstens zwei anhand der Emissionsspektren ausgewählte unterschiedliche Stoffe auf. Dadurch kann ein Analyseergebnis hoher Aussagekraft erzeugt werden, wenn die Stoffe beispielsweise Lichtmerkmale wie die Spezies erzeugen, welche in dem Gewebe vermutet werden, welches der Behandlung unterzogen werden soll, und/oder wenn sich aus Genauigkeit, mit welcher Lichtmerkmale und/oder Signaturen daraus aus der Testlichterscheinung, zu welcher die Stoffe beitragen, bestimmt werden können, aus welcher Genauigkeit auf die Genauigkeit, mit welcher die Lichtmerkmale und/oder Signaturen der Eingriffslichterscheinung und/oder der Spezies, welche zu der Eingriffslichterscheinung beitragen, geschlossen werden kann.

Das Testobjekt kann eine vorbestimmte Zusammensetzung aufweisen. Bevorzugt ist das Testobjekt, insbesondere die Zusammensetzung und/oder Gestalt der Testoberfläche, anhand der klinischen Indikation für den operativen Eingriff beispielsweise der (vermuteten) Krankheit des (vermuteten) Tumors und/oder des zu behandelnden Gewebes ausgewählt.

Bevorzugt enthält das Testobjekt gezielt Stoffe, welche auch in dem zu untersuchenden und/oder zu behandelnden Gewebe vorkommen und/oder vermutet werden, um eine Testlichterscheinung zu erzeugen, aus welcher Lichtmerkmale bestimmt werden können, welche auch aufgrund der Lichterscheinung (Eingriffslichterscheinung), erzeugt durch Einwirkung des Instruments auf das Gewebe beim HF-chirurgischen Eingriff generiert werden können.

Bevorzugt ist die Elektrode, die der Anwender zur Erzeugung der Testlichterscheinung in die Nähe der Gegenelektrode bringt, gezielt aufgrund ihrer Zusammensetzung für die Erzeugung der Testlichterscheinung ausgewählt. Beispielsweise kann die Elektrode anhand des zu erwartenden Beitrags des Stoffes oder Materials der Elektrode zu der Testlichterscheinung und/oder der Lichterscheinung und/oder anhand eines oder mehrerer Lichtmerkmale der Testlichterscheinung und/oder der Lichterscheinung, welche Lichtmerkmale aufgrund der Zusammensetzung der Elektrode vorhanden sind, ausgewählt werden. Die Elektrode kann hinsichtlich zu erwartender Beiträge zu der Testlichterscheinung und/oder der Lichterscheinung aufgrund der Zusammensetzung der Elektrode in Abhängig von der klinischen Indikation für einen operativen Eingriff ausgewählt sein.

Das Testobjekt kann wenigstens zwei unterschiedliche Testoberflächen aufweisen. Die Testoberflächen können sich hinsichtlich ihrer Gestalt, Zusammensetzung, Aggregatzustand (fest, flüssig) usw. unterscheiden. Die Zusammensetzungen der Testoberflächen können sich insbesondere in der Art der Spezies und/oder deren absoluten und/oder relativen Mengen unterscheiden. Damit kann der Einfluss der Elektrode und/oder des Elektrodenmaterials ermittelt und/oder aus einem Analyseergebnis der Testlichterscheinung und/oder der Eingriffslichterscheinung herausgerechnet werden. Die Testoberflächen können sich dadurch unterscheiden, dass diese aus unterschiedlichen Materialien bestehen. Beispielsweise können die Testoberflächen aus unterschiedlichen Metallen, insbesondere Reinmetallen oder Legierungen, unterschiedlichen Lösungen, in denselben oder unterschiedlichen Materialen, beispielsweise Schwamm oder Filzmaterial, enthalten. Der Test kann beinhalten, dass eine Testlichterscheinung nacheinander gegen wenigstens zwei unterschiedliche Testoberflächen erzeugt wird.

Die Testeinrichtung ist dazu eingerichtet, aus bestimmten Lichtmerkmalen der Testlichterscheinung eine Kalibrierung der Analyseeinrichtung zu bestimmen. Beispielsweise können die bestimmten Lichtmerkmale mit in einer Datenbank hinterlegten Lichtmerkmalen verglichen werden, um die Kalibrierung der Analyseeinrichtung zu bestimmen. Bevorzugt wird aufgrund der Kalibrierung wenigstens ein Parameter der Testeinrichtung, der Analyseeinrichtung, insbesondere der Lichtaufnahmeeinheit und/oder der Analyseeinheit, des HF-chirurgischen Instruments und/oder des HF-chirurgischen Geräts ausgewählt oder verändert. Bevorzugt ist das System dazu eingerichtet, aufgrund der Kalibrierung - wenigstens einen Parameter der Testeinrichtung, der Analyseeinrichtung, insbesondere der Lichtaufnahmeeinheit und/oder der Analyseeinheit, des HF-chirurgischen Instruments und/oder des HF-chirurgischen Geräts oder einer sonstigen Einrichtung oder Komponente des Systems auszuwählen oder zu verändern.

Bevorzugt wird von der Testeinrichtung aufgrund des Tests mit der Testeinrichtung anhand der Testlichterscheinung wenigstens ein Wert oder ein Wertebereich wenigstens eines Parameters, beispielsweise eines Steuer- und/oder Auswerteparameters, der Testeinrichtung, des Testobjekts, der Analyseeinrichtung und/oder der HF-Chirurgieeinrichtung, insbesondere des HF-chirurgischen Instruments und/oder eines HF-chirurgischen Geräts zur Speisung des HF-chirurgischen Instruments, vorgegeben. Insbesondere kann aufgrund des Tests mit der Testeinrichtung anhand der Testlichterscheinung ein Wert oder Wertebereich eines Parameters wenigstens einer der genannten Einrichtungen des Systems vorgegebene werden, welcher Einfluss auf den Betrieb des Systems aus den genannten Einrichtungen bei der Behandlung des Gewebes hat. Dies wäre selbstverständlich bevorzugt ein Wert oder Wertebereich eines Parameters, welcher das gewünschte HF-chirurgische Behandlungsergebnis ermöglicht, aber zu einer verbesserten Analyse der Eingriffslichterscheinung mittels der Analyseeinrichtung führt.

Das Testobjekt ist vorzugsweise dazu eingerichtet, einen Mindestabstand zwischen einer Elektrode, welche vom Anwender der Testeinrichtung zur Erzeugung der Testlichterscheinung in die Nähe der Gegenelektrode gebracht wird, und einer Testoberfläche des Testobjekts festzulegen, zwischen denen die Testlichterscheinung erzeugt werden soll.

Bevorzugt ist die Testeinrichtung dazu eingerichtet, die Atmosphäre, insbesondere deren Zusammensetzung und/oder Druck, zwischen einer Elektrode und der Testoberfläche und/oder Gegenelektrode festzulegen. Beispielsweise kann zwischen der Elektrode und dem Testobjekt eine Argonatmosphäre erzeugt werden.

Das Testobjekt ist vorzugsweise dazu eingerichtet, eine Ausrichtung einer Elektrode, welche zur Erzeugung der Testlichterscheinung in die Nähe der Gegenelektrode gebracht wird, und/oder einer Lichtaufnahmeeinheit relativ zu der Testoberfläche festzulegen. Beispielsweise kann dies geschehen, indem das Testobjekt dazu eingerichtet ist, das Instrument und/oder die Lichtaufnahmeeinheit beim Einführen des Instruments und/oder der Lichtaufnahmeeinrichtung in das Testobjekt zu führen, so dass das Instrument und/oder die Lichtaufnahmeeinrichtung eine Lage automatisch einnimmt, so dass das Instrument und/oder die Lichtaufnahmeeinrichtung die festgelegte Ausrichtung relativ zu der Gegenelektrode haben.

Die Testeinrichtung stellt bevorzugt eine Abschirmung der Lichtaufnahmeeinheit gegen Umgebungslicht bei der Aufnahme des Lichts der Testlichterscheinung bereit.

Die zuvor genannten Maßnahmen dienen jede für sich oder in beliebiger (Unter-)Kombination dazu, Bedingungen für den Test zu schaffen, um ein besonders aussagekräftiges Testergebnis zu erhalten.

Bevorzugt ist die Testeinrichtung für ein Instrument und eine Analyseeinrichtung bestimmt, welche vom Anwender des Instruments, z.B. einem Chirurgen, einem Operationsassistenten oder einem sonstigen Mitglied des Operationsteams, zur Vorbereitung einer Operation mit dem Instrument oder während der Operation mit dem Instrument kombinierbar sind. Instrument und Lichtaufnahmeeinheit der Analyseeinrichtung sind von dem Anwender des Instruments vor der Operation und/oder während der Operation bevorzugt aneinander befestigbar, um die Lage der Lichtaufnahmeeinheit der Analyseeinrichtung relativ zu dem Instrument festzulegen. Gerade bei einem solchen Operationssystem aus Instrument und Analyseeinrichtung, welche aneinander von dem Anwender des Instruments befestigbar sind, kann es aufgrund einer Fehlbedienung, Beschädigung, Verschmutzung, einem Nichtzusammenpassen des Instruments und der Analyseeinrichtung zu einer nicht funktionsfähigen Analyseeinrichtung kommen und/oder einer Analyseeinrichtung, welche einer besonderen Einstellung der HF-Chirurgieeinrichtung und/oder der Analyseeinrichtung bedarf, so dass die Analyseeinrichtung arbeiten kann. Solche Fälle können aufgrund des Tests mit der Testeinrichtung aufgedeckt und korrigiert werden.

Erfindungsgemäß wird folglich ein System angegeben, welches eine erfindungsgemäße Testeinrichtung und eine Vorrichtung zur Befestigung einer Lichtaufnahmeeinheit einer Analyseeinrichtung an dem Instrument oder an einer Komponente des Instruments aufweist. Die Vorrichtung ist bevorzugt derart einrichtet, dass die Lichtaufnahmeeinheit durch den chirurgischen Anwender eines chirurgischen Instruments oder seinen Assistenten mit dem Instrument verbindbar ist, besonders bevorzugt während einer Operation.

Die Vorrichtung kann beispielsweise ein Adapter sein in dem oder an dem eine optische Faser als Lichtaufnahmeeinheit befestigt oder befestigbar ist. Der Adapter kann mit dem Instrument, beispielsweise einem Handgriff des Instruments, verbunden werden, um eine Lage der Lichtaufnahmeeinheit relativ zu einer Elektrode des Instruments festzulegen. Mittels der Testeinrichtung kann überprüft werden, ob die so mit dem Instrument kombinierte Lichtaufnahmeeinheit korrekt arbeiten kann.

Das erfindungsgemäße Verfahren ist ein Verfahren zum Kalibrieren einer Analyseeinrichtung zur Analyse von Lichterscheinungen, welche Lichterscheinungen durch ein HF-chirurgisches Instrument bei der Einwirkung auf biologisches Gewebe erzeugt werden. Das Verfahren beinhaltet das Erzeugen einer Testlichterscheinung zwischen einer Elektrode und einem Testobjekt, welches kein menschlicher oder tierischer Körper ist, und das Aufnehmen von Licht der Testlichterscheinung durch eine Lichtaufnahmeeinheit. Das Verfahren wird also insbesondere nicht am menschlichen oder tierischen Körper durchgeführt. Das Verfahren kann mit einer hierin beschriebenen Testeinrichtung und/oder einem hierin beschrieben System mit einer Testeinrichtung und einer HF-Chirurgieeinrichtung durchgeführt werden. Insbesondere können einzelne oder mehrere beliebige Verfahrensschritte von oder mittels einer hierin beschriebenen Testeinrichtung und/oder einem hierin beschriebenen System durchführt werden. Der Anwender der Analyseeinrichtung kann das Testen, insbesondere um sich von der Funktionsfähigkeit der Analyseeinrichtung zu überzeugen oder diese zu justieren, durch Erzeugen einer Testlichterscheinung mittels des dazu zweckbestimmten Testobjekts durchführen, anstatt ein Objekt dazu Zweck zu entfremden oder den Test gar am Patienten durchzuführen.

Das Verfahren kann zur Vorbereitung einer Operation unmittelbar vor der Operation oder während der Operation durchgeführt werden, und zwar durch den Anwender des Instruments, beispielsweise einen Chirurgen oder einen Operationsassistenten. Die Testeinrichtung, insbesondere das Testobjekt, ist bevorzugt zur Verwendung durch einen Arzt, welcher mit dem HF-chirurgischen Instrument eine angesetzte (geplante) Operation durchzuführen beabsichtigt, oder einen Assistenten des Arztes zur Vorbereitung der Operation und/oder während der Operation eingerichtet.

In einem Ausführungsbeispiel des Verfahrens kann je wenigstens eine Testlichterscheinung zwischen einer Elektrode und wenigstens zwei unterschiedlichen Testoberflächen erzeugt und ausgewertet werden.

Bevorzugt wird zum Testen das System mit HF-chirurgischem Instrument, HF-chirurgischem Gerät und Analyseeinrichtung verwendet, welches auch für die Operation verwendet wird.

Bevorzugt werden zum Testen die gleichen Einstellungen für das Instrument vorgenommen oder erfolgt das Testen mit den gleichen Einstellungen, welche auch für die Operation mit dem Instrument verwendet werden sollen und/oder davon abgeleitete Einstellungen.

Weitere vorteilhafte Merkmale und Ausführungsformen ergeben sich aus den Unteransprüchen sowie folgender Beschreibung und den Figuren, welche schematisch und beispielhaft zeigen:
Figur 1a - eine Veranschaulichung der Positionierung einer Lichtaufnahmeeinheit relativ zu einer Elektrode bzw. einer Lichterscheinung positionierten Lichtaufnahmeeinheit beim Einwirken der Elektrode auf Gewebe,
Figur 1b - eine schematische Veranschaulichung der Elektrodenspitze im Lichtakzeptanzkegel einer optischen Faser,
Figur 2a - ein beispielhaftes Instrument in Form eines Handgriffs mit distal angeordneter Elektrode, welcher in einen Adapter eingesteckt ist, welcher einer Lichtaufnahmeeinheit in Form einer optischen Faser aufweist,
Figur 2b - ein Ausschnitt einer Kombination aus einem Instrument und einem Adapter wie in Figur 2a mit einer anderen Elektrodenform,
Figur 3 - eine beispielhafte Ausführungsform eines Systems mit einer HF-chirurgischen Einrichtung mit einer Analyseeinrichtung und einer Testeinrichtung,
Figur 4 - zeigt eine weitere Ausführungsform eines Systems mit einer HF-Chirurgieeinrichtung, einer Analyseeinrichtung sowie einer Testeinrichtung,
Figur 5 - ein Ausführungsbeispiel eines Testobjekts,
Figur 6a - ein weiteres Ausführungsbeispiel eines Testobjekts,
Figur 6b1 und 6b2 - je eine weitere beispielhafte Gegenelektrode und Elektrode,
Figur 6c - eine beispielhafte Gegenelektrode und
Figur 7 - ein Ausführungsbeispiel eines erfindungsgemäßen Verfahrens.

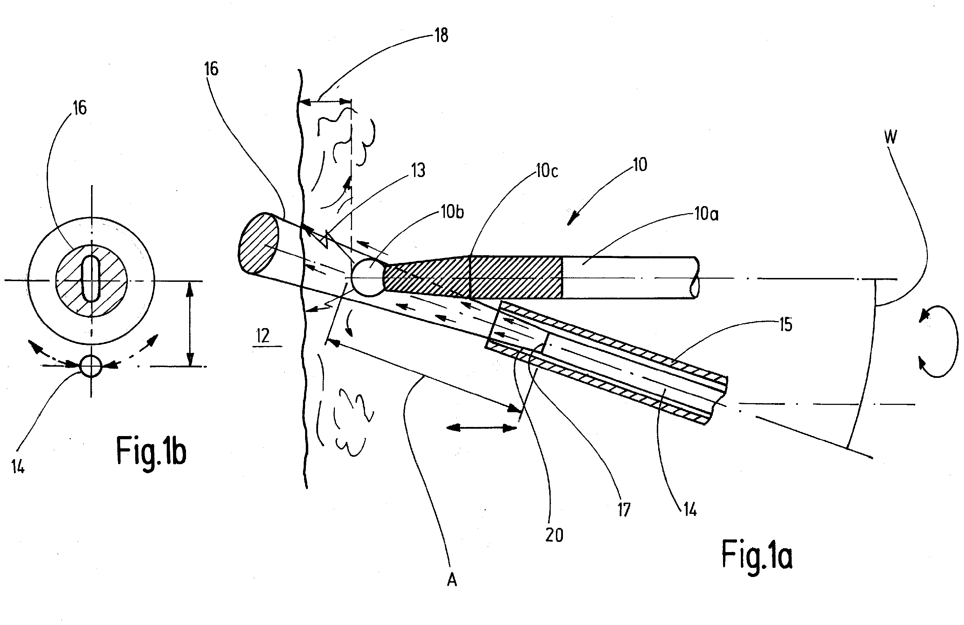

Figur 1a zeigt eine Elektrode 10 wie sie beispielhaft in einem chirurgischen Instrument 11 verwendet sein kann. Die Elektrode 10 ist an zu behandelnden Gewebe 12 eines Patienten angeordnet. Mittel zum Halten der Elektrode 10, wie z.B. ein Griffstück, sind in Figur 1a der Übersichtlichkeit halber nicht gezeigt. Die Elektrode 10 weist einen Schaft 10a und einen Kopf oder Spitze 10b auf, welche beispielsweise aus Titan oder Wolfram bestehen können. Auf der Elektrode 10 ist angrenzend an den Kopf oder die Spitze 10b eine Isolation 10c angebracht, um die Bereiche der Elektrode 10 genauer festzulegen, zwischen denen und dem Gewebe 12 ein Plasma 13, insbesondere Funken oder Lichtbögen, erzeugt wird. Die Elektrode 10, die bei der Erzeugung der Plasmaeingriffslichterscheinung 13 verwendet wird, ist vorzugsweise eine teilisolierte Schneideelektrode wie sie in der Hochfrequenz(HF)-Chirurgie verwendet wird. Die Elektrode 10 weist bevorzugt eine Abbrandfestigkeit gegen Funken von wenigstens der von Titan oder Wolfram auf.

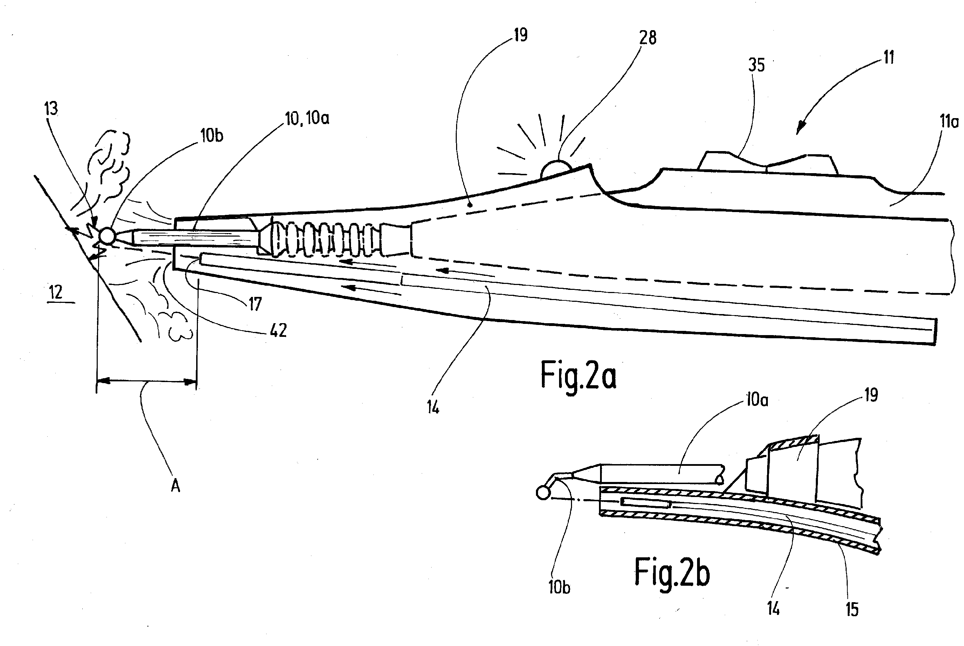

Unter einem spitzen Winkel W zu der Elektrode 10 ist eine optische Faser 14 in einem Schlauch 15 oder eine Leitung 15 angeordnet. Die optische Faser 14 und Schlauch 15 sind in ihrer Lage relativ zu der Elektrode 10 fixiert. Es ist ein Lichtakzeptanzkegel 16 eingezeichnet, welcher alle Richtungen enthält, aus denen die optische Faser 14 Licht aufnehmen und zu einer Analyseeinheit (in Figur 1a und 2 nicht dargestellt) zur Analyse des Lichts weiterleiten kann. Wie dargestellt, ist die Spitze oder der Kopf 10b der Elektrode 10 innerhalb des Lichtakzeptanzkegels 16 angeordnet. Dies ist auch in der schematischen Schnittdarstellung durch den Lichtakzeptanzkegel 16 in Figur 1b erkennbar. Zwischen der Elektrode 10 und dem Gewebe 12 auftretende Lichterscheinungen 13, wie etwa ein stehendes Plasma wie bei der Argonplasmakoagulation, ein Lichtbogen, oder, ggf. in rascher Folge, entstehende und vergehende Funken, entstehen damit innerhalb des Lichtakzeptanzkegels 16. Deren Licht, welches infrarote, sichtbare und/oder ultraviolette elektrische Wellen aufweist, wird damit teilweise von der optischen Faser 14 aufgenommen und zu der Analyseeinheit weitergeleitet.

Der Schlauch 15 dient der Leitung eines Fluids, insbesondere Gases, beispielsweise CO₂, zum distalen Ende des Schlauches 15, wo dieses in Richtung zu der Elektrode 10 austritt. Der Fluidfluss dient der Reduzierung der Verschmutzung der optischen Faser 14, dem Reinigen der optischen Faser 14, dem Wegblasen von entstehendem Dampf oder Partikeln, aufgrund der elektrochirurgischen Einwirkung auf das Gewebe 12.

Es ist jedoch möglich, dass sich trotz des austretenden CO₂-Flusses, welcher in seiner Strömungsrate auf vorzugsweise maximal 1 Liter/Minute begrenzt ist, um zu behandelndes Gewebe 12 nicht zu verdrängen, Partikel oder Dämpfe auf dem Lichteingang 17 der optischen Faser 14 abscheiden und die Transmissivität der optischen Faser 14 einschränken. Diese Einschränkung kann möglicherweise auch nur für bestimmte Wellenlängen, z.B. im infraroten, sichtbaren und/oder ultravioletten Bereich des elektromagnetischen Spektrums zu verzeichnen sein. Durch Verschmutzung der Elektrode 10 kann der Lichteingang 17 der optischen Faser 14 teilweise abgeschattet werden. Durch Verschmutzung der Elektrode 10 kann sich die Form der Lichterscheinung 13 teilweise verändern, denn durch die Verschmutzung kann ein Teil der Spitze oder des Kopfes 10b elektrisch isoliert werden. Verschmutzungen können nicht nur auf Grund von Dämpfen oder wegfliegenden Partikeln auftreten. Es ist auch möglich, dass der Chirurg versehentlich das Gewebe 12 mit der Elektrode 10 berührt. Denn der Zündabstand, welcher zum Zünden der Lichterscheinung zwischen Elektrode 10 und Gewebe 12 höchstens bestehen darf, kann beispielsweise lediglich wenige Millimeter zwischen dem distalen Ende der Elektrode 10 und dem Gewebe 12 betragen, wenn die Lichterscheinung in einer mit Edelgas, z.B. Argon angereicherten Umgebung gezündet wird, oder sogar nur wenige zehntel Millimeter, wenn die Lichterscheinung in einer mit CO₂ angereicherten Umgebung gezündet wird. Verschmutzungen können auch zu einer Veränderung des Spektrums der Lichterscheinungen führen.

Wie viel Licht von der Lichterscheinung 13 mittels der optischen Faser 14 eingefangen werden kann, hängt stark von deren Lage relativ zu der Elektrode 10 ab. Beispielsweise hängt dies von dem Abstand A des Lichteingangs 17 von der Spitze der Elektrode 10 und/oder von der Lichterscheinung 13, der Anordnung des Lichteingangs 17 um die Elektrode 10 herum und dem Winkel W zwischen der optischen Faser und der Elektrode 10 usw. ab.

Figur 2a zeigt ein HF-chirurgisches Instrument 11 in Form eines HF-Applikators mit einem Handgriff 11a und einer vorzugsweise auswechselbaren Elektrode 10. Der Applikator 11 steckt in einem Adapter 19. Alternativ können Applikator 11 und Adapter 19 beispielsweise nebeneinander angeordnet aneinander fixierbar sein. Der Adapter 19 enthält eine darin oder daran fixierte optische Faser 14 und kann einen Kanal 20 zum Ausgeben eines CO₂-Stroms bereitstellen. Der Adapter 19 dient dazu, die optische Faser 14 und das Instrument 11 aneinander zu befestigen. Der Chirurg oder ein anderes Mitglied des Operationsteams kann den Adapter 19 beispielsweise während der Operation mit dem Instrument 11 verbinden.

Der Adapter 19 unterstützt die Festlegung der Lage der optischen Faser 14 relativ zu der Elektrode 10. Jedoch bleiben viele Variationsmöglichkeiten übrig. Beispielsweise ist die Elektrode 10 vorzugsweise austauschbar. Unterschiedliche Formen der Spitze oder des Kopfes 10b der Elektrode 10 sind möglich. Beispielsweise sind nadelförmige und spatelförmige Elektroden 10 bekannt. In Figur 2b ist beispielsweise eine hakenförmige Elektrode 10 anstatt der Elektrode 10 dargestellt in Figur 2a eingesetzt. Auch können die Elektroden 10 unterschiedliche Längen aufweisen und damit unterschiedlich weit aus dem Adapter 19 herausschauen. Von der Form und der Länge der aus dem Adapter 19 herausschauenden Länge der Elektrode 10 hängt aber die Lage, d.h. der Abstand und/oder die Orientierung des Lichteingangs 17 relativ zu der Elektrode 10 ab, und damit auch die Lage der Lichterscheinung 13 relativ zu dem Lichtakzeptanzkegel 16.

Die Festlegung der Lage der optischen Faser 14 relativ zu der Elektrode 10 ist dann besonders zuverlässig, wenn der HF-Applikator 11 zu dem Adapter 19 passt. Jedoch ist es denkbar, dass versehentlich oder beabsichtigt ein HF-Applikator 11 verwendet wird, zu welchem der Adapter 19 nicht passt. Dann ist eine korrekte Lage der optischen Faser 14 relativ zu der Elektrode 10 möglicherweise nicht mehr sichergestellt.

Zu erwähnen ist außerdem, dass die Lichtaufnahmeeinheit, beispielsweise die optische Faser 14, beschädigt sein kann. Auch können Kopplungsstücke zwischen der Lichtaufnahmeeinheit 14 und der Analyseeinheit oder bei einer mehrteiligen Lichtaufnahmeeinheit 14 zwischen Teilen der Lichtaufnahmeeinheit 14 ebenfalls defekt sein und zu einer beeinträchtigten Übertragung von Licht führen.

Die Elektrode 10 bzw. das Instrument 11 werden von einem HF(Hochfrequenz)-Generator (in Figur 1a und 2 nicht gezeigt) mit elektrischer Energie gespeist. Bei HF-Generatoren lässt die Norm eine Standardabweichung der HF-Ausgangsleistung von +/- 20% zu. Je nach Art des gewünschten HF-chirurgischen Eingriffs, beispielsweise Schneiden, Koagulieren, Devitalisieren, Thermofusionieren, usw. werden unterschiedliche Leistungen, Wellenformen der HF-Spannung, mit welcher die Elektrode 10 gespeist wird, usw. benötigt. Die Eigenschaften der HF-Spannung, mit welcher die Elektrode 10 gespeist wird, werden als Mode oder Betriebsart bezeichnet werden. Die Betriebsart hat Einfluss auf die Lichtemission der HF-Lichterscheinung 13, beispielsweise des HF-Funkens, am biologischen Gewebe 12.

Hinzu kommt, dass der spezifische elektrische Widerstand von biologischem Gewebe 12 stark variiert. Fettgewebe beispielsweise hat etwa 3,3·10⁷ Ohm mm²/m, Muskelgewebe 2,0·10⁶ Ohm mm²/m, und Blut dagegen nur 1,6·10⁶ Ohm mm²/m. Auch kann das Medium (Aerosol, Luftgemisch) zwischen Elektrode 10 und Gewebe 12 stark von dem Einsatz abhängen. Je nachdem ob der Eingriff offenchirurgisch, endoskopisch oder laparoskopisch durchgeführt wird, kann die Feuchtigkeit zwischen Elektrode 10 und Gewebe 12 beispielsweise stark variieren. Die Zündfähigkeit (Ionisation) eines Plasmas 13 wird sehr stark von der Feuchtigkeit oder Flüssigkeit an der aktiven Elektrode 10 beeinflusst. Die Atmosphäre zwischen der Elektrode 10 und dem Gewebe 12 kann teilweise von der HF-Chirurgieeinrichtung bestimmt werden, je nachdem ob beispielsweise Argongas oder CO₂-Gas in den Bereich zwischen Elektrode 10 und Gewebe ausgegeben wird. Die Durchschlagsspannung ist von der Gasart abhängig. Wird beispielsweise der Funken 13 in der Argonatmosphäre gezündet, ist dieser anders als wenn der Funke 13 in trockener Luft oder in einer CO₂-Atmosphäre gezündet wird.

Zusammenfassend lässt sich feststellen, dass die Art der Lichterscheinung 13 oder deren Lichtmerkmale, insbesondere deren Form, Intensität, spektrale Zusammensetzung usw. sehr stark von den individuellen Gegebenheiten für einen konkreten operativen Eingriff abhängen und sich im Verlaufe des Eingriffs ändern können. Wie viel, auch wellenlängenabhängig, von dem Licht erzeugt durch die Lichterscheinung 13 durch die optische Faser 14 aufgefangen und/oder in ein elektrisches Signal in der Analyseeinrichtung umgesetzt werden kann, hängt ebenfalls stark von den Gegebenheiten bei der Operation ab.

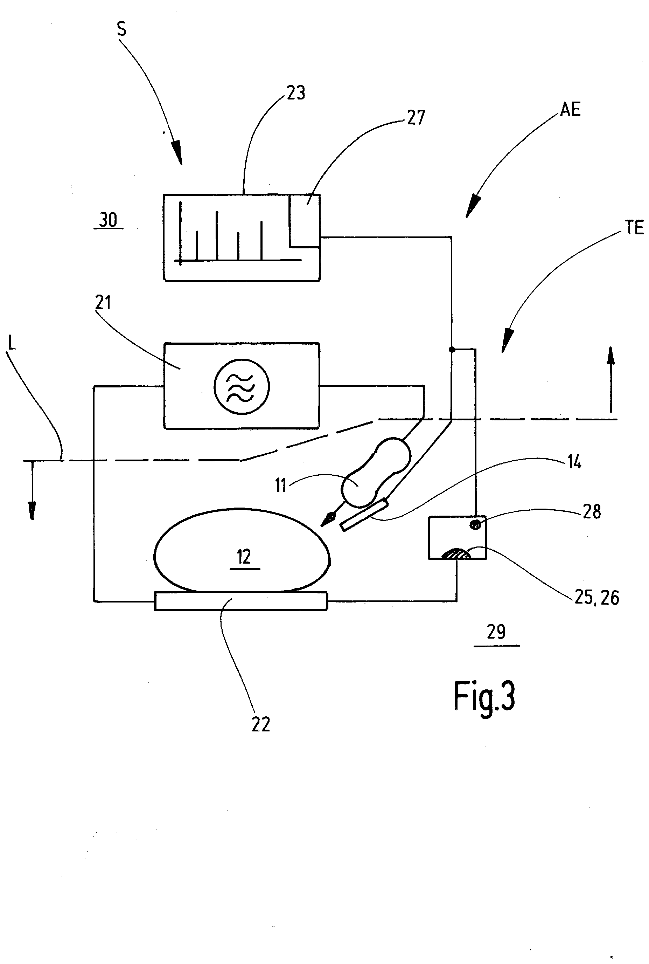

Ein erfindungsgemäß verbessertes Konzept für ein System S zur HF-chirurgischen Behandlung (insbesondere Schneiden und/oder Koagulieren) von Gewebe 12 unter Einsatz von optischer Emissionsspektroskopie ist in Figur 3 beispielhaft und schematisch dargestellt. Das System S (HF-Chirurgiesystem) weist eine HF-Chirurgieeinrichtung E mit einem HF-chirurgischen Instrument 11, hier in Form eines Applikators 11 mit einem Handgriff 11a und einer am distalen Ende des Handgriffes 11a befestigten Elektrode 10, und einem HF-Generator 21 zur Speisung des Instruments 11 auf. Die HF-Chirurgieeinrichtung E ist eine Einrichtung zur monopolaren HF-Chirurgie, bei welcher der Stromkreis über eine Neutralelektrode 22 geschlossen wird, welche am Patienten befestigt ist. Alternativ oder zusätzlich ist die HF-Chirurgieeinrichtung E für die bipolare HF-Chirurgie eingerichtet.

Das System S weist zudem eine Analyseeinrichtung AE zur Analyse der Lichterscheinungen, insbesondere stehendes Plasma, Lichtbögen, Funken, zwischen der Elektrode 10 und dem Gewebe 12 des Körpers des Patienten mittels optischer Emissionsspektroskopie im infraroten, sichtbaren und/oder ultravioletten Bereich des Spektrums auf. Die Analyseeinrichtung AE weist eine optische Faser 14 als Lichtaufnahmeeinheit und eine Analyseeinheit 23 auf, die mit der optischen Faser 14 verbunden ist. Die optische Faser 14 kann entsprechend Figur 1a mittels eines Adapters 19 an dem Instrument 11 fixiert sein. Das mittels der optischen Faser 14 von der Stelle, an der der Eingriff stattfindet, übertragene Lichtsignal wird von dem System S in ein elektrisches Signal umgesetzt.

Die Testeinrichtung TE zum Kalibrieren der Analyseeinrichtung AE weist ein Testobjekt 24 auf, welches eine Gegenelektrode 25 mit einer Testoberfläche 26 bereitstellt. Mittels des Instruments 11 kann eine Plasmatestlichterscheinung in Form beispielsweise eines Funkens oder eines fortwährend unterhaltenen Plasmas zwischen dem Instrument 11 und dem Testobjekt 24 erzeugt werden. Das Testobjekt 24 ist insofern zum Erzeugen einer Plasmatestlichterscheinung eingerichtet. Die Gegenelektrode 25 kann auf demselben elektrischen Potential liegen, wie die Neutralelektrode 22. Die Gegenelektrode 25 kann beispielsweise mit dem HF-Generator 21 über die Neutralelektrode 22 verbunden sein. Die Testeinrichtung TE weist zudem eine Auswerteeinheit 27 auf. Diese Auswerteeinheit 27 kann mit der Analyseeinheit 23 in ein Gerät integriert oder Teil der Analyseeinheit 23 sein. Die Analyseeinheit 23 und/oder Auswerteeinheit 27 können in verschiedenen Geräten integriert sein. Die Analyseeinheit 23 und/oder die Auswerteeinheit 27 können auch über mehrere Geräte verteilt sein und beispielsweise durch ein oder mehrere Softwaremodule gebildet sein, welche in einem Gerät oder in mehreren Geräten ausgeführt werden. Die Testeinrichtung TE kann zudem eine Signalisierungseinheit 28 zur optischen, akustischen und/oder haptischen Ausgabe einer Meldung an den Anwender des Systems S aufweisen. Die Signalisierungseinheit 28 ist mit der Auswerteeinheit 27 verbunden, um von dieser gegebenenfalls zur Abgabe der Meldung veranlasst zu werden.

Durch eine gestrichelte Linie L ist der sterile Bereich 29 des Operationssaals von einem nicht sterilen Bereich getrennt eingezeichnet. Wie dargestellt ist die Testeinrichtung TE teilweise im sterilen Bereich 29 angeordnet. Insbesondere ist das Testobjekt 24 innerhalb des sterilen Bereichs 29 angeordnet.

Mittels der Testeinrichtung TE gemäß Figur 3 lässt sich überprüfen, ob das System S, insbesondere die Analyseeinrichtung AE, funktionsfähig ist und/oder lässt sich das System S, insbesondere die Analyseeinrichtung AE einstellen, um mit dem System S bzw. der Analyseeinrichtung AE arbeiten zu können. Insbesondere lässt sich vorzugsweise überprüfen, ob mittels der Analyseeinrichtung AE eine Gewebedifferenzierung mittels optischer Emissionsspektroskopie an Lichterscheinungen hervorgerufen durch Einwirkung mittels einer Elektrode 10 auf Gewebe 12 durchgeführt werden kann. Alternativ lässt sich mit der Testeinrichtung TE mittels eines Tests eine Einstellung für das System S, insbesondere die Analyseeinrichtung AE, finden, so dass diese mit einer vorgegebenen Genauigkeit arbeiten kann.

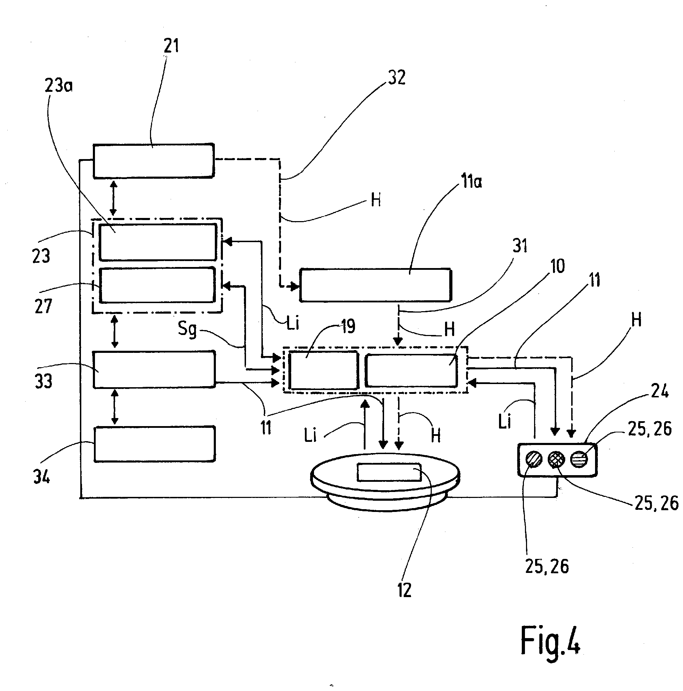

Figur 4 veranschaulicht ein System S für die HF-Chirurgie unter Anwendung der optischen Emissionsspektroskopie. Bei der Ausführungsform gemäß Figur 4 kann es sich um eine Konkretisierung des Systems S gemäß Figur 3 oder um eine andere Ausführungsform des erfindungsgemäßen Systems S handeln. In Figur 4 sind Ströme von Medien, Leistungen und Signalen eingezeichnet (HF-Strom H, Licht Li, Statussignal Sg und Spülmedium M (z.B. CO₂)). Die HF-Elektrode 10 wird über eine Leitung 31 in dem Handgriff, welcher über eine Leitung 32 mit dem HF-Chirurgiegerät 21 verbunden ist, mit elektrischer HF-Energie gespeist. Zwischen der HF-Elektrode 10 und dem Körper 12 des Patienten fließt ein HF-Strom. Dabei wird eine Lichterscheinung 13 erzeugt, wobei deren Licht von der optischen Faser 14 aufgenommen und einer Analyseeinheit 23 zugeführt wird. Über ein Spülsystem 33 wird Spülmedium, insbesondere CO₂ an die Eingriffstelle zwischen Elektrode 10 und Gewebe ausgegeben. Das Spülsystem 33 ist mittels eines Fußschalters 34 steuerbar. Die HF-Chirurgieeinrichtung E ist mittels Tasten 35 (s. Figur 2a) am Handgriff 11a und/oder am Chirurgiegerät 21 steuerbar.

Im Falle eines Tests mit der Testeinrichtung TE fließt ein Strom zwischen der Elektrode 10 und einer Testoberfläche 26. Spülmedium kann ausgegeben werden, um eine Atmosphäre zwischen der Elektrode 10 und der Testoberfläche 26 festzulegen. Licht eines Lichtfunkens wird von der Lichtaufnahmeeinheit 14 aufgenommen und an die Analyseeinheit 23 weitergeleitet. Die Analyseeinheit 23 kann ein Statussignal an den HF-Applikator 11 oder den Adapter 19 ausgeben.

Wie in Figur 4 gezeigt, kann die Testeinrichtung TE mehrere Testoberflächen 26 aufweisen. Die Testoberflächen 26 können sich voneinander unterscheiden. Die Testoberflächen 26 können sich insbesondere in ihrer Zusammensetzung, Form, Größe, Phase der Testoberfläche 26 (z.B. flüssig, fest) usw. unterscheiden. Jede Testoberfläche 26 kann eine Legierung oder ein Reinmetall (Reinheit größer oder gleich 95%) aufweisen. Die Legierungen der unterschiedlichen Testoberflächen 26 können sich voneinander unterscheiden und/oder die Reinmetalle, welche in den unterschiedlichen Testoberflächen 26 enthalten sind, können sich voneinander unterscheiden.

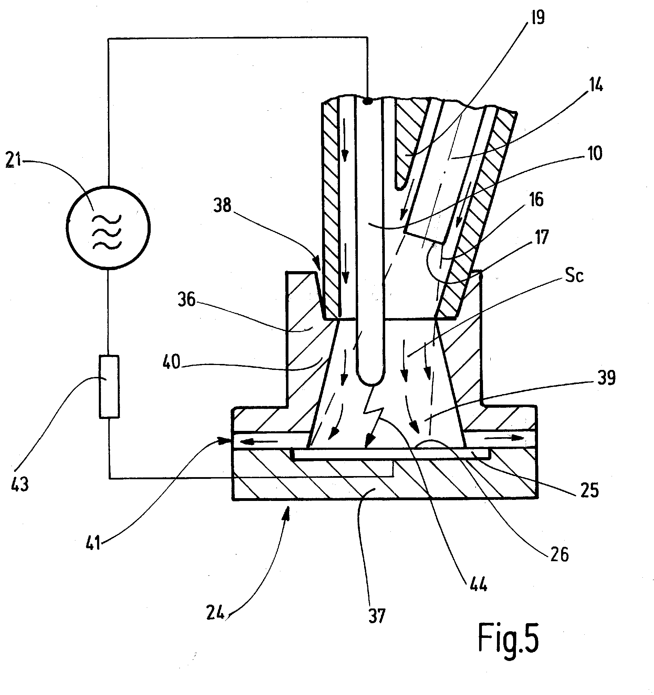

Figur 5 zeigt ein beispielhaftes Testobjekt 24 in einer Schnittdarstellung. Das Testobjekt 24 kann beispielsweise in dem System gemäß Figur 3 oder 4 verwendet werden. Das Testobjekt 24 weist einen Abstandshalter 36 und einen Träger 37 der Testoberfläche 26 auf. Die Testoberfläche 26 wird von eine Referenzelektrode oder Gegenelektrode 25 bereitgestellt. Die Gegenelektrode 25 kann beispielsweise eine Metallplatte oder eine Metallfolie sein.

Der Abstandshalter 36 stellt eine Öffnung 38 bereit, in die der Adapter bzw. das Instrument 11 eingeführt oder auf welche oder an welche der Adapter bzw. das Instrument 11 angesetzt werden kann. Aufgrund der Abstandshalterfunktion ist sichergestellt, dass der Abstand zwischen dem Instrument 11, dem Handgriff und/oder dem Adapter und der Testoberfläche 26 einen Maximalwert nicht überschreitet.

Bevorzugt ist die Öffnung 38 dazu eingerichtet, das Instrument 11, den Adapter 19 und/oder die Elektrode 10 und/oder die Lichtaufnahmeeinrichtung 14 zu führen, um eine bestimmte Lage der Elektrode 10 und/oder der Lichtaufnahmeeinrichtung 14 relativ zu der Gegenelektrode 25 festzulegen. Bevorzugt sind die Form der Öffnung 38 einerseits und die Form des Instruments 11, der Elektrode 10, der Lichtaufnahmeeinrichtung 14 und/oder des Adapters 19 andererseits auf einander abgestimmt, so dass, wenn die Formen miteinander in Eingriff stehen, die Lage des Instruments 11, der Lichtaufnahmeeinheit 14, der Elektrode 10 und/oder des Adapters 19 relativ zu der Testoberfläche 26 festgelegt ist.

Zwischen der Öffnung 38 des Abstandshalters 36 und der Testoberfläche 26 ist ein Raum 39 (Plasmaraum) für das Plasma gebildet. Der Raum 39 wird von elektrisch isolierenden Wänden 40 (bis auf die Gegenelektrode 25) gebildet. Das Testobjekt 24 weist wenigstens eine Auslassöffnung 41 auf, durch die Gas, welches aus dem Adapter in den Raum 39 strömt, ausgegeben werden kann, so dass ein bestimmter Druck an Schutzgas Sc, beispielsweise CO₂ oder Argon, in dem Raum 39 herrscht oder einen bestimmter Druck nicht überschritten wird. Zum Beispiel kann die Leitung 15 in dem Adapter 19 für den Test mit Argon gespeist werden. Zudem können sorgt die Auslassöffnung 41 für eine gleichbleibende Atmosphäre durch fortwährenden Austausch des Mediums in dem Raum 39. In Figur 5 erkennbar steht die Elektrode 10, welche zu dem HF-Instrument 11 in Kombination mit der Lichtaufnahmeeinrichtung 14 gehört, über das distale Ende des Instruments 11 bzw. des Adapters 19 in den Raum 39 für das Plasma über. Der Lichteingang 17 der optischen Faser 14 ist gegenüber dem Ausgang 42 (s. Figur 2a) des Adapters 19 bzw. des Instruments 11 zurückversetzt.

Die Gegenelektrode 25 kann über einen Vorwiderstand 43 mit dem HF-Generator 21 verbunden sein. Die Elektrode 10 ist ebenfalls mit dem HF-Generator 21 verbunden, um von diesem mit HF-Energie gespeist zu werden. Damit wird die Lichterscheinung (Testlichterscheinung) 44 zwischen der Spitze 10b der Elektrode 10 und der Gegenelektrode 25 erzeugt.

Das Material für die Gegenelektrode 25 bzw. die Testoberfläche 26 kann gezielt aufgrund der klinischen Indikation für den HF-chirurgischen Eingriff ausgewählt werden. Beispielsweise kann das Material Spezies enthalten, welche auch in dem zu behandelnden Gewebe 12, insbesondere zu schneidenden und/oder zu koagulierenden Gewebe 12 vermutet werden, welches bei dem HF-chirurgischen Eingriff behandelt wird.

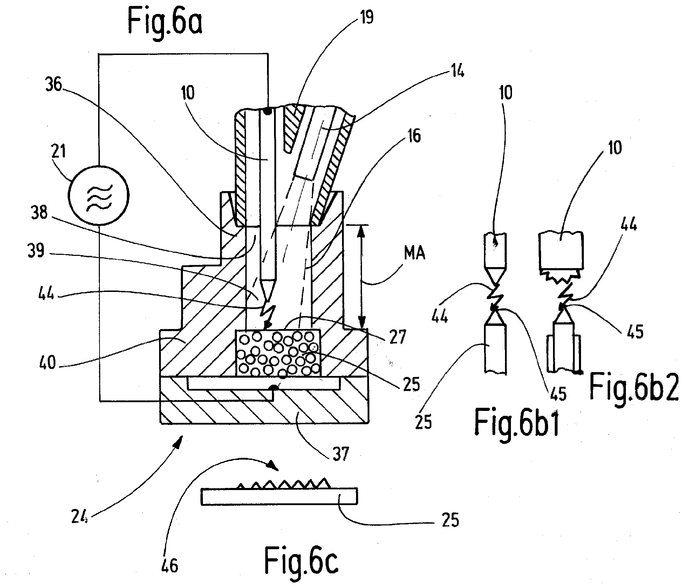

Figur 6a zeigt eine alternative Ausführungsform eines Testobjekts 24. Das Testobjekt 24 kann beispielsweise in dem System gemäß Figur 3 oder 4 verwendet werden. Die Testoberfläche 26 wird hier von einem Schwamm oder Vlies 25 getränkt mit einer Referenzlösung, insbesondere Salzlösung, gebildet. Damit kann eine Testoberfläche 26 geschaffen werden, die der des realen Gewebes 12 nahe kommt. In die Lösung können gezielt Stoffe eingebracht sein, die mit der Testeinrichtung TE testweise nachgewiesen und/oder differenziert werden sollen. Die Lösung und/oder die Stoffe können gezielt auf Grund der klinischen Indikation für den HF-chirurgischen Eingriff ausgewählt sein. Damit kann beispielsweise eine Differenzierung von Spezies und damit eine "Gewebe"differenzierung testweise nicht am Körper des Patienten, sondern an einem körperfremden Material durchgeführt werden. Die Referenzlösung kann gezielt aufgrund der medizinischen Indikation für den chirurgischen Eingriff ausgewählt sein, etwa weil die bestimmte Referenzlösung Spezies enthält, die auch oder in der konkreten Zusammensetzung auch in dem Gewebe 12 erwartet werden, welches beim HF-chirurgischen Eingriff teilweise in den Plasmazustand überführt wird und deswegen Lichtmerkmale zu der Eingriffslichterscheinung 13 beiträgt.

Die Testoberfläche 26 kann, wie in den Beispielen gemäß Figur 5 und Figur 6a, eine im Wesentlichen ebene Fläche sein. Die Gegenelektrode 25 kann alternativ wie in den Ausführungsbeispielen gemäß Figur 6b1 und 6b2 eine Spitze 45 aufweisen. Die Elektrode 10 kann beispielsweise eine runde Spatelform (Figur 5), eine spitze Form (Figur 6a, Figur 6b1) oder eine Spatelform mit spitzen Formen (Figur 6b2) aufweisen. Die Elektrode 10 kann eine Isolierung aufweisen, welche die Spitze oder den Kopf der Elektrode 10 unisoliert lässt. Figur 6b3 zeigt ein Beispiel einer Gegenelektrode 25 als Metallplatte mit Rillen 46. Mit der Elektrodenform und der Gegenelektrodenform lässt sich die Form der Testlichterscheinung 44 vorbestimmen, um zu einem aussagekräftigen Testergebnis zu gelangen.

Die Gegenelektrode 25 ist bevorzugt abbrandfest, wenn diese für den Mehrmalgebrauch gedacht ist. Bevorzugt ist die Abbrandfestigkeit jedoch geringer als die der Elektrode 10. Die Testeinrichtung TE ist bevorzugt sterilisierbar. Die Testeinrichtung TE, insbesondere das Testobjekt 24 kann zum Mehrmalgebrauch ausgelegt sein. Ein Testobjekt 24 bzw. eine Gegenelektrode 25 als Einmalprodukt wird bevorzugt mittels eines getränkten Schwammes oder Vlieses 25 gebildet.

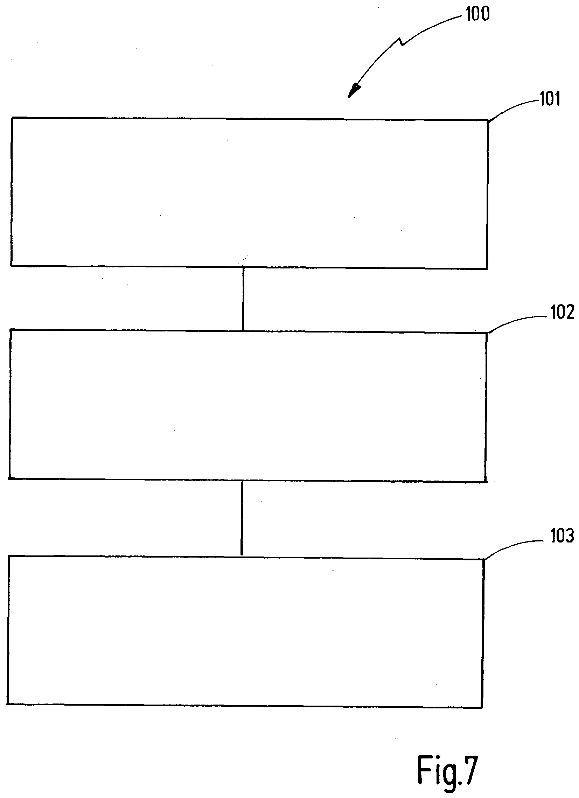

Mittels des erfindungsgemäßen Systems S gemäß wenigstens eines der beschriebenen Ausführungsbeispiele kann beispielsweise wie folgt gearbeitet werden:
Das System S gemäß beispielsweise Figur 3 oder 4 kann erst von dem Operationsteam für den geplanten Eingriff zusammengestellt worden sein. Insbesondere kann das Instrument 11 und/oder die Elektrode 10 und/oder das Testobjekt 24 und/oder die Testoberfläche 26 oder Gegenelektrode 25 für den Eingriff auf Grundlage der klinischen Indikation ausgewählt worden sein. Zur Vorbereitung des Eingriffs oder während des Eingriffs kann der Anwender der Analyseeinrichtung AE einen Funktionstest der Analyseeinrichtung AE durchführen, indem der Anwender mittels des Instruments 11 eine Testlichterscheinung 44 zwischen der Elektrode 10 des Instruments 11 und der Gegenelektrode 25 erzeugt (Schritt 101 des Ausführungsbeispiels des Verfahrens 100 gemäß Figur 7). Mögliche Ergebnisse des Funktionstest können beispielsweise "arbeitet" oder "arbeitet nicht" sein. Der Anwender kann das Instrument 11 dazu in die Öffnung 38 des Testobjekts 24 einführen oder auf die Öffnung 38 aufsetzen. Die Testeinrichtung TE kann dazu eingerichtet sein, zu erkennen, wenn das Instrument 11 mit dem Testobjekt 24 zum Zweck des Erzeugens einer Testlichterscheinung 44 zusammengebracht wird. Die Testeinrichtung TE kann beispielsweise dazu eingerichtet sein, zu erkennen, wenn das Instrument 11 bzw. der Adapter 19 in die Öffnung 38 eingeführt oder aufgesetzt werden. Die Testeinrichtung TE kann auf Grund des Erkennens eines beabsichtigten Tests vorzugsweise bestimmte Einstellungen des Systems S, insbesondere der HF-Chirurgieeinrichtung E, auswählen. Beispielsweise kann die Testeinrichtung TE bestimmte ansonsten mögliche Einstellungen des Systems S für den Test ausschließen oder ansonsten nicht mögliche Einstellungen ermöglichen. Alternativ oder zusätzlich ist die Testeinrichtung TE dazu eingerichtet, dass das Operationsteam mittels eines Bedienelements (nicht gezeigt) das System S in einen Testmodus schalten kann. Der Anwender kann das Erzeugen der Testlichterscheinung 44 selbst auslösen oder die Testeinrichtung TE kann den Test automatisch auslösen. Die Testeinrichtung TE kann Mittel (nicht gezeigt) zum Erkennen einer Position der Elektrode 10, des Instruments 11 und/oder des Adapters an dem Testobjekt 24 oder in dem Testobjekt 24 aufweisen. Damit kann beispielsweise automatisch festgestellt werden, ob die Elektrode 10, das Instrument 11 oder der Adapter in einer Entfernung zu der Testoberfläche 26 angeordnet ist, so dass die Erzeugung einer Testlichterscheinung 44 automatisch ausgelöst oder die manuelle Auslösung freigegeben wird. Die Testeinrichtung TE ist kann dazu eingerichtet sein, aufgrund den Voraussetzungen des Tests (z.B. verwendete Elektrode 10, Instrument 11, HF-Chirurgiegerät 21, Art der Gegenelektrode 25 usw.) automatisch eine geeignete Einstellung zur Erzeugung der Testlichterscheinung auszuwählen. Die Testeinstellungen bzw. die Testeinstellmöglichkeiten sind so gewählt, dass die HF-Elektrode 10 oder die optische Faser 14 bei dem Test nicht beschädigt werden. Die HF-Spannung zwischen der Elektrode 10 und der Gegenelektrode 25 beispielsweise ist vorzugsweise wählbar. Diese hängt insbesondere von der Form der Elektroden 10, vom Abstand der Elektroden 10 zueinander, vom Werkstoff der Elektrode 10 und der Gegenelektrode 25 und von dem Gas um die Elektrode 10 und die Gegenelektrode 25 in dem Testobjekt 24 ab. Alternativ oder zusätzlich kann für die Erzeugung der Testlichterscheinung 44 im Wesentlichen dieselbe Einstellung des Systems S verwendet werden, wie für den eigentlichen HF-chirurgischen Eingriff.

Durch das Auslösen des Tests entsteht eine Plasmatestlichterscheinung 44, beispielsweise ein stehendes Plasma wie bei der Argonplasmakoagulation, ein Lichtbogen oder ein Funken, zwischen der Elektrode 10 des Instruments 11 und der Gegenelektrode 25, indem ein Hochspannungsimpuls die Atmosphäre zwischen Elektrode 10 und Testoberfläche 26 ionisiert und damit elektrisch leitfähig macht. Dadurch wird die Strecke niederohmig und es kann sich ein Stromfluss von mehreren Ampere ausbilden. Bei der Funkenanregung beispielsweise wird der Stromfluss nach wenigen Mikrosekunden wieder unterbrochen, wird jedoch nach der Pausenzeit wieder neu gestartet. Die Funkenfolge hängt von der Wiederholfrequenz des Generators ab, z.B. 20 kHz. Die Stromform der Spannungsquelle kann ein reiner Sinus von z.B. 350 kHz oder eine modulierte (Sinus)spannungsform sein. Es kann ein Einzelpuls, ein Pulspaket mit mehreren Schwingungen, die jeweils mit einer Wiederholfrequenz versehen sind, so dass z.B. ein Plasma aufrecht erhalten bleibt, verwendet werden. Beim Erzeugen der Testlichterscheinung 44 oder der Eingriffslichterscheinung 13 (so kann die Lichterscheinung hervorgerufen auf Grund des eigentlichen Eingriffs auf das Gewebe 12 des Patienten mit der Elektrode 10 bezeichnet werden) kann eine Spannungs-, Leistungs- oder Plasmaregelung verwendet werden. Bei der Plasmaregelung (auch Lichtbogenregelung genannt) werden ein oder mehrere Merkmale des Plasmas, z.B. bestimmte Oberwellen, konstant oder innerhalb eines Bereichs gehalten.

Das Licht der Testlichterscheinung 44 wird von der Lichtaufnahmeeinrichtung 14 teilweise aufgenommen (Schritt 102) und an eine Vorrichtung 23a (siehe Figur 4) der Analyseeinheit 23 weitergeleitet, welche dazu eingerichtet ist, das Licht, welches durch die optisch Faser 14 weitergeleitet wird, in ein elektrisches Signal umzusetzen, welches der weiteren Auswertung durch die Auswerteeinheit 27 der Testeinrichtung TE zugeführt wird. Die Vorrichtung 23a kann das Licht beispielsweise wie ein Spektrometer wellenlängenspezifisch in einzelne Beiträge zu einem oder mehreren elektrischen Signalen umsetzen.

Um die Funktionsfähigkeit der Analyseeinrichtung AE zu bewerten kann beispielsweise die Signalleistung auf Grund der Testlichterscheinung 44 ermittelt werden, welche Signalleistung an der Auswerteeinheit 27 ankommt. Die Auswerteeinheit 27 kann beispielsweise die Leistung, beispielsweise die Gesamtleistung oder wellenlängenspezifische Intensitäten, mit Referenzwerten vergleichen, um die Übertragung des Lichts der Testlichterscheinung 44 bis zur Vorrichtung 23a zum Umsetzen des Lichtsignals in ein elektrisches Signal zu überprüfen. Dabei kann der Energieeintrag aufgrund der HF-Spannung und des HF-Stroms zum Erzeugen der Testlichterscheinung 44 berücksichtigt werden.

Der Test ist dann besonders aussagekräftig, wenn mittels des Testobjekts 24 die Elektrode 10 relativ zu der Testoberfläche 26 bzw. Gegenelektrode 25 ausgerichtet wird und/oder die Lage der Elektrode 10 relativ zu der Testoberfläche 26 und/oder Gegenelektrode 25 festgelegt wird. Alternativ oder zusätzlich kann mittels des Testobjekts der Adapter 19 relativ zu der Testoberfläche 26 bzw. Gegenelektrode 25 ausgerichtet werden und/oder die Lage des Adapters 19 relativ zu der Testoberfläche 26 und/oder Gegenelektrode 25 festgelegt werden. Sollte die Testlichterscheinung beispielsweise zu schwach sein oder erst gar nicht zünden, kann dies auf eine ungeeignete Lage der optischen Faser 15 relativ zu der Elektrode 10 hindeuten. Dies ist ein Beispiel dafür, dass mittels der Testeinrichtung TE Bedienfehler oder sonstige Fehler bei der Kombination der optischen Faser 15 mit dem Instrument 11 oder eine für die Lichtanalyse ungeeignete Bestückung des Instruments 11 mit einer Elektrode 10 aufgedeckt werden können.

Wenn das Testobjekt 24 dazu eingerichtet ist, Fremdlicht aus der Umgebung bei dem Test von dem Lichteingang 17 der Lichtaufnahmeeinheit 14 fernzuhalten, kann die Aussagekraft des Tests zusätzlich oder alternativ erhöht werden. Auf Grund dessen wird das Umgebungslicht nicht mitaufgenommen, wobei dies bedeutet, dass allenfalls eine Lichtmenge aus der Umgebung aufgenommen wird, welche den Test mit der Testeinrichtung TE nicht beeinträchtigt. Alternativ oder zusätzlich kann die Testeinrichtung TE auf eine Operationsfeldbeleuchtung (nicht dargestellt) abgestimmt sein. Die Analyseeinheit 23 und/oder die Auswerteeinheit 27 sind vorzugsweise in demjenigen Spektralbereich unempfindlich, in dem die Operationsfeldbeleuchtung arbeitet. Auf diese Weise wird eine Blendung der Auswerteeinheit 27 und/oder der Analyseeinheit 23, insbesondere deren Diskriminatoreinheit (nicht gezeigt) vermieden, welche Diskriminatoreinheit der Zuordnung von Lichtmerkmalen zu Gewebemerkmalen dient, um eine Gewebeunterscheidung mittels der Analyseeinrichtung AE durchführen zu können.

Für eine definierte Plasmaerzeugung, insbesondere Funkenanregung, ist eine definierte Atmosphäre, in welcher die Anregung erfolgt, wie einer Argongasatmosphäre mit hoher Reinheit, von Vorteil. Wenige PPM-Sauerstoff sowie Wasserdampf oder Dämpfe organischer Verbindungen im Argon, die beim biologischen Gewebe 12 immer vorhanden sind, können die Entladung empfindlich stören. Deshalb wird mittels der Testeinrichtung TE vorzugsweise Atmosphäre, in welcher das Plasma zur Erzeugung der Testlichterscheinung 44 gezündet wird vorzugsweise festgelegt. Dies betrifft insbesondere die Zusammensetzung und/oder den Druck der Atmosphäre. Das Testobjekt 24 stellt einen Zündraum (Plasmaraum) 39 bereit. Beispielsweise wird während der Durchführung des Tests der Plasmaraum 39 in dem Testobjekt 24 mit Gas, beispielsweise CO₂ und/oder Argon geflutet. In dem Zündraum 39 kann ein konstanter Druck bereitgestellt werden. Wenn die Funkenerzeugung unter Schutzgasbedingungen (z.B. Argongas) durchgeführt wird, können dadurch andere Einflüsse aus der Umgebung (Betriebsmikroklima) weitgehend unterbunden werden. Durch Edelgas, z.B. Argon, kann sich die Signalstärke der Lichtemission positiv verstärken. Das Testobjekt 24 kann beispielsweise einen Anschluss (nicht gezeigt) zur Speisung des Plasmaraums 39 mit Argon aufweisen, so dass beispielsweise für das Erzeugen der Testlichterscheinung 44 der Plasmaraum mit Edelgas, insbesondere Argon, flutbar ist oder geflutet wird, wohingegen die Spüleinrichtung beispielsweise andernfalls insbesondere beim HF-chirurgischen Eingriff, mit CO₂ arbeitet.

Gemäß dem erfindungsgemäßen Konzept gemäß kann die konkrete Analyseeinrichtung AE getestet werden, welche zur Analyse bei dem chirurgischen Eingriff verwendet werden soll oder verwendet wird. Insbesondere kann die konkrete Lichtaufnahmeeinheit 14 und die Analyseeinheit 23 auf Funktionsfähigkeit überprüft werden und zwar anhand einer Plasmatestlichterscheinung 44, zwischen der Elektrode 10, welche beim Eingriff verwendet wird, und einer Gegenelektrode 25 ohne Einwirkung auf den Körper des Patienten. Die Testbedingungen kommen also dem eigentlichen Einsatz auf Grund der Verwendung weitgehend identischer Komponenten sowie ebenso wie beim eigentlichen Eingriff einem auf Grund der Beaufschlagung einer Elektrode 10 eines Instruments 11 gezündeten Plasma sehr nahe. Mittels des Tests kann eine ungeeignete Lage der optischen Faser 14 bezüglich der Elektrode 10, z.B. durch unkorrekten Sitz des Handgriffs im Adapter, die Verwendung einer ungeeigneten Elektrode 10, einer verschmutzte oder beschädigte optischen Faser 14, eine unzureichende Kopplung zwischen Abschnitten der optischen Faser 14 oder zwischen optischer Faser 14 und Vorrichtung 23a zur Umwandlung des Lichtsignals in ein elektrisches Signal ausgeschlossen werden. Auch kann eine Abschattung auf Grund einer möglicherweise verschmutzten Elektrode 10, eine ungünstige Form der Testlichterscheinung 44 (und damit möglicherweise der Eingriffslichterscheinung 13) auf Grund einer Verschmutzung oder eine ungünstige Elektrodenform ausgeschlossen werden. Mit der Testeinrichtung TE kann überprüft werden, ob die Signalstärke für eine aussagekräftige Auswertung des aufgenommenen Lichtsignals bei der Analyse der Lichtmerkmale, hervorgerufen durch die Eingriffslichterscheinung 13 aufgrund des HF-Eingriffs am Gewebe 12, ausreicht und benutzt werden kann.

Zur Anregung der Testlichterscheinung 44 kommt bevorzugt das HF-Chirurgiegerät 21 der HF-Chirurgieeinrichtung E zum Einsatz, welches die Elektrode 10 auch beim Eingriff am Gewebe 12 des Patienten speist. Dazu werden die zu analysierende Probe (Testoberfläche 26, Gegenelektrode 25) und die HF-Elektrode mit dem Generator 21 verbunden.

Die Testeinrichtung TE kann dem chirurgischen Anwender mittels der Signalisierungseinheit ein Statussignal (z.B. "Analyseeinrichtung funktionsfähig" oder "Analyseeinrichtung nicht funktionsfähig") optisch, akustisch und/oder fühlbar übermitteln.

Testeinrichtung TE kann dazu eingerichtet sein, dem Anwender ggf. Maßnahmen zu empfehlen, um eine Verbesserung der Analyse durch die Analyseeinrichtung AE zu ermöglichen, insbesondere um ein stärkeres Signal zu erhalten. Die Testeinrichtung TE kann dafür beispielsweise eine Wahrscheinlichkeit für eine oder mehrere Einschränkungen ermitteln, dass diese für das, möglicherweise negative, Testergebnis verantwortlich sind.

Mittels des Testverfahrens 100 kann im Rahmen einer Kalibrierung eine Wellenlängenabhängigkeit der Transmissivität der Lichtaufnahmeeinheit 14 festgestellt und gegebenenfalls können eine oder mehrere Korrektur(faktor)en ermittelt werden, welche bei der Auswertung des Signals aufgrund der Testlichterscheinung 44 und/oder des Signals aufgrund der Eingriffslichterscheinung 13 berücksichtig werden können. Das Feststellen, das Ermitteln und/oder das Berücksichtigen können von der Testeinrichtung TE und/oder der Analyseeinrichtung AE automatisch vorgenommen werden.

Um die Wellenlängenabhängigkeit der Transmissivität zu bestimmen, wird bevorzugt eine Testlichterscheinung 44 erzeugt, in deren Spektrum wenigstens zwei oder mehr Spektrallinien vorhanden sind und/oder es werden mehrere Testlichterscheinungen 44 erzeugt, in deren Spektrum je wenigstens eine Spektrallinie vorhanden ist, wobei sich wenigstens eine Spektrallinie von einer Testlichterscheinung 44 zur anderen Testlichterscheinung 44 unterscheiden. Solche Testlichterscheinungen 44 können gezielt erzeugt werden, wenn die Zusammensetzung der Testoberfläche 26 anhand der Art der Spezies in der Zusammensetzung - und damit der charakteristischen Spektrallinien - sowie deren Häufigkeit entsprechend ausgewählt ist.

Aufgrund des Plasmas, das zum Erzeugen der Testlichterscheinung zwischen der Elektroden 10 und der Gegenelektrode 25 erzeugt wird, wird Material der Gegenelektrode 25 und/oder der Elektrode 10 abgebaut (verdampft, atomisiert und ionisiert) und es werden damit Spezies (Atome, Moleküle, deren Ionen) aus dem Material der Gegenelektrode 25 und der Elektrode 10 erzeugt, die nun auch in dem Plasma vorkommen. Die Energiezufuhr bewirkt, dass diese Spezies Strahlung abgeben. Es wird Licht mit einem Spektrum mit Anteilen im infraroten, sichtbaren und/oder ultravioletten Bereich erzeugt, welche Anteile bezüglich der Lage und Intensität der Spektrallinien für die betreffende Spezies charakteristische Spektrallinien aufweisen.

Dies wird von der Analyseeinrichtung AE zur Gewebedifferenzierung verwendet, denn wenn beim chirurgischen Eingriff ein Plasma zwischen Elektrode 10 und Gewebe 12 gezündet wird, werden Spezies des Gewebematerial in das Plasma aus dem Gewebe 12 herausgelöst und treten in das Plasma ein, wobei die Spezies auf Grund dessen einen charakteristischen Beitrag zum Spektrum des Lichts der Eingriffslichterscheinung 13 leisten. Für jede neue klinische Indikation sind meist unterschiedliche Spurenelemente ausschlaggebend, um eine Gewebedifferenzierung während der Operation durchführen zu können.

Von Vorteil ist daher, wenn die Zusammensetzung der Testoberfläche 26 bzw. der Gegenelektrode 25 speziell auf Grund der klinischen Indikation für den Eingriff, insbesondere auf Grund der erwarteten Zusammensetzung des Gewebes 12, welches HF-chirurgisch behandelt werden soll, und/oder die Zusammensetzung gezielt auf Grund des Emissionsspektrums bei Überführung von Material aus der Gegenelektrode 25 in die Plasmaphase ausgewählt ist. Als Material für die Gegenelektrode 25 bzw. die Testoberfläche 26 können Stoffe, beispielsweise ein oder mehrere Metalle (insbesondere Reinmetall oder eine Legierung) oder eine Flüssigkeit (Referenzlösung) mit damit enthaltenen ausgewählten Spezies (Referenzsubstanzen), ausgewählt werden, welche auch charakteristisch in dem zu untersuchenden biologischen Gewebe 12 vorkommen und/oder vermutet werden, z.B. Ca, K, Mg, Na, P, Cl, Cd, Zn, Rb, Cr, Co, Fe, I, Cu, Mn, Mo, Se, F, Si, As, Ni, Sn und V. Damit kann eine Testlichterscheinung 44 erzeugt werden, aus welcher Lichtmerkmale ermittelt werden können, welche auch auf Grund der Eingriffslichterscheinung 13, erzeugt durch Einwirkung des Instruments 11 auf das Gewebe 12 generiert werden können. Das Testobjekt 24 kann beispielsweise durch das OP-Team gezielt auf Grund der klinischen Indikation ausgewählt sein.

Das Testobjekt 24 kann zwei unterschiedliche Testoberflächen 26 aufweisen, die sich in ihrer Zusammensetzung unterscheiden. Es können beispielsweise mehrere Metallplättchen mit Testoberflächen 26 aus unterschiedlichem Reinmetall vorhanden sein. Damit kann der Einfluss des Materials der Elektrode 10 herausgerechnet werden, denn auch das Elektrodenmaterial kann einen Beitrag zum Spektrum der Testlichterscheinung 44 und/oder der Eingriffslichterscheinung 13 leisten.

Auch die Elektrode 10 kann gezielt auf Grund ihrer Zusammensetzung für die Erzeugung der Testlichterscheinung 44 ausgewählt werden, insbesondere anhand der zu erwartenden Lichtmerkmale, welche auf Grund des Stoffes der Elektrode 10 zu der Testlichterscheinung 44 und/oder der Eingriffslichterscheinung 13 beigetragen werden. Das Spektrum der Beiträge des Materials der Elektrode 10 zu der Testlichterscheinung 44 und/oder deren Lichtmerkmale können beispielsweise als Referenzspektrum oder Referenzlichtmerkmale verwendet werden. Dies ist insbesondere von Bedeutung, wenn die Elektrode 10 nach ihrem Material speziell für die klinische Indikation ausgewählt ist, die bei der folgenden Operation mit dem System S durchgeführt werden soll. Die Spektrallinien des Materials der Elektrode 10 überlagern bevorzugt nicht die Spektrallinien des zu untersuchenden Gewebes 12 und/oder des Materials der Gegenelektrode 25, um Testergebnisse und/oder Spektroskopieergebnisse - gewonnen anhand des Lichts der Testlichterscheinung 44 oder der Eingriffslichterscheinung 13 - nicht zu verfälschen. Der Elektrodenwerkstoff und das Material der Testoberfläche 26 und/oder der Gegenelektrode 25 können unterschiedlich sein, um eine eindeutige Zuordnung von Lichtmerkmalen oder Signaturen oder Spurenelementen durchführen zu können.

Der Test auf Funktionsfähigkeit der Analyseeinrichtung AE kann zusätzlich oder alternativ zu dem oben beschrieben Test zur Ermittlung der Signalstärke eine Ermittlung der Genauigkeit, beispielsweise des Signal-zu-Rauschen-Verhältnisses, über das gesamte Spektrum der Testlichterscheinung 44 hinweg, über ausgewählte Bereiche des Spektrums und/oder bei einer oder mehreren bestimmten Spektrallinien beinhalten, mit welcher Genauigkeit die Signalintensität auf Grund der Testlichterscheinung 44 bei einer oder mehreren Wellenlängen bestimmt werden kann. Vorzugsweise wird die Genauigkeit für eine oder mehrere Spektrallinien ermittelt, welche sowohl im Spektrum der Testlichterscheinung 44 als auch im Spektrum der Eingriffslichterscheinung 13 vorkommen. Denn ein mögliches Verfahren zur Gewebedifferenzierung, welches mittels der Analyseeinheit 23 angewendet wird, basiert auf der Ermittlung einer Signatur aus Lichtmerkmalen des Spektrums des Lichtes der Eingriffslichterscheinung 13 und der Ermittlung von Gewebemerkmalen, welche zu der Signatur geführt haben, auf Grund der Signatur. Zur Unterscheidung zwischen gesundem Gewebe 12 und pathologischem Gewebe 12 beispielsweise kann jede Signatur aus Intensitätsverhältnissen ausgewählter, für verdampfte Teile des biologischen Gewebes 12 kennzeichnender Spektrallinien berechnet sein. Anhand der ermittelten Genauigkeit kann von der Testeinrichtung TE ermittelt werden, ob eine Gewebedifferenzierung über die Analyseeinrichtung AE möglich ist.

Zusätzlich oder alternativ kann testweise eine Differenzierung eines oder mehrerer Stoffe durchgeführt werden, welche gezielt in einer oder mehreren Testoberflächen 26 verwendet werden. Die Testeinrichtung TE kann beispielsweise dazu eingerichtet sein, Signaturen aus Lichtmerkmalen des Lichts der Testlichterscheinung 44 zu ermitteln, die aus Intensitäten mehrerer Wellenlängen oder Teilspektren berechnet sind, wobei jede Signatur bevorzugt aus Intensitätsverhältnissen ausgewählter für in den Plasmazustand übergegangene Teile des Materials der Testoberfläche 26 und/oder der Elektrode 10 und/oder der Atmosphäre zwischen der Elektrode 10 und der Testoberfläche 26 kennzeichnende Spektrallinien berechnet ist. Die Testeinrichtung TE kann Mittel (nicht dargestellt) zum Empfangen von Informationen über die Zusammensetzung der Testoberflächen 26 aufweisen, um der Testeinrichtung TE solche Informationen zu übermitteln. Die Testeinrichtung TE kann das Differenzierungsergebnis auf Grund der Analyse des Lichts der Testlichterscheinung mit der Information vergleichen, welche die Testeinrichtung TE über das Mittel zum Empfangen von Informationen über die Zusammensetzung der Testoberflächen 26 erhalten hat.

Auf Grund des Testergebnisses kann die Testeinrichtung TE ein Signal über das Testergebnis an den Anwender ausgeben und/oder eine oder mehrere mögliche Maßnahmen identifizieren, wie das die Genauigkeit zu erhöhen ist oder eine Gewebedifferenzierung verbessert werden kann, z.B. durch Austausch oder Reinigung einer oder mehrerer ausgewählter Komponenten des Systems S.

Bei der Ermittlung eines Verhältnisses zwischen Intensitäten von Spektrallinien in dem Licht der Testlichterscheinung 44 und/oder der Eingriffslichterscheinung 13 kann die aus der Testlichterscheinung 44 ermittelte Wellenlängenabhängigkeit der Transmissivität mittels eines Korrekturfaktors berücksichtigt werden.

Das System S, insbesondere die Analyseeinrichtung AE, kann mittels des Spektrums der Testlichterscheinung 44 kalibriert werden, um damit feste Einflussfaktoren auf die Transmission des Lichtes durch den Eingang der optischen Faser 14 bis zu der Einheit mittels der das Licht in ein elektrisches Signal umgewandelt wird, berücksichtigen zu können. Bei zu starken Abweichungen der Lichtleistung nach oben oder nach unten könnte dies vom System S erkannt und z.B. über die Anpassung der Integrationszeit oder z.B. durch einen Korrekturfaktor der Analyseeinheit 23 kompensiert werden. Eine Kalibrierung des gesamten Systems S mit allen relevanten Baugruppen, die Einfluss auf die Signalgüte eines Lichtfunkens haben können, kann somit erfasst und angepasst werden. Mit der Testeinrichtung TE kann das System S kalibriert werden. Unter einer Kalibrierung wird verstanden, dass die Genauigkeit des Systems S im Rahmen eines Tests ermittelt wird und dass bevorzugt aufgrund des Tests mit der Testeinrichtung TE wenigstens eine Einstellung oder ein Parameter des Systems S, insbesondere der Analyseeinrichtung AE und/oder der HF-Chirurgieeinrichtung E, gefunden und eingestellt wird, um eine gewünschte Genauigkeit mit der Analyseeinrichtung AE zu erreichen.

Ein Testergebnis, welches aus dem Licht aufgenommen aus der Testlichterscheinung 44 ermittelt wurde, kann vor oder während der Operation gewonnen werden und als Referenz für den restlichen Operationseinsatz gespeichert werden. Mittels der Referenz, insbesondere eines Referenzsignals, können beispielsweise Spektrallinien im Licht, hervorgerufen durch die Eingriffslichterscheinung 13 zwischen Elektrode 10 und Gewebe 12, identifiziert werden.

Die Testeinrichtung TE sollte vorteilhaft im sterilen Bereich 29 eingesetzt werden können, damit eine Überprüfung der Transmission auch nach einer Anwendung während der Operation möglich wird. Vorteil für den Anwender ist die Funktionsüberprüfung des Systems S vor oder nach einer Anwendung direkt am OP-Tisch. Die Testeinrichtung TE kann zur Überprüfung der Signalgüte einer Lichterscheinung, insbesondere eines Lichtfunkens zur Gewebedifferenzierung für ein System und/oder zur Überprüfung der Analyseeinrichtung AE zur optischen Emissionsspektroskopie eingesetzt werden. Die Testeinrichtung TE kann zur Überprüfung der Signalgüte oder der Funkengüte oder der voraussichtlichen Funkengüte eines Lichtfunkens oder der Analyseeinrichtung AE vor und nach Anwendung einer Gewebedifferenzierung eingesetzt werden.

Auf Grund des Test kann die Testeinrichtung TE automatisch eine oder mehrere Einstellungen für den Test finden. Beispielsweise kann auf Grund der Auswertung des Testlichterscheinung ermittelt werden, dass die Einstellung des HF-Generators zur Erzeugung der Testlichterscheinung 44 angepasst werden müssen, um eine Testlichterscheinung 44 zu erzeugen, auf Grund welcher eine genauere Aussage über die Funktionsfähigkeit der Analyseeinrichtung AE getroffen werden kann und der Test kann automatisch mit der angepassten Einstellung wiederholt werden. Die von dem HF-Generator 21 abgegebene Spannung ist veränderbar, um zu verhindern, dass die Elektrode 10 bei der Funkenerzeugung abbrennt. Zudem ist die von dem HF-Generator 21 abgegebene Spannung bevorzugt veränderbar derart, dass die gewünschten Elemente angeregt werden und sich die Spektrallinien ausreichend vom Grundrauschen in der Intensität abheben.

Auf Grund des Tests kann die Testeinrichtung TE automatisch eine oder mehrere Einstellungen für das System S für den Eingriff festlegen oder Einstellungsmöglichkeiten bestimmen, insbesondere einschränken. Die einen oder mehreren Einstellungen oder Einstellungsmöglichkeiten werden bevorzugt auf Grund des Testergebnisses gewonnen aus der Auswertung der Testlichterscheinung 44 ermittelt. Das System S kann dazu eingerichtet sein, auf Grund der Auswertung des Signals auf Grund des Lichts der Testlichterscheinung 44 eine Einstellung oder mögliche Einstellungen für das Erzeugen der Eingriffslichterscheinung 13, das Aufnehmen des Lichts der Eingriffslichterscheinung 13, das Umsetzen des aufgenommenen Lichts der Eingriffslichterscheinung 13 in ein elektrisches Signal und/oder die Analyse des Signals festzulegen. Auf Grund des Testergebnisses kann also von der Testeinrichtung TE gezielt Einfluss auf die Erzeugung und/oder Analyse des Signals auf Grund der Lichterscheinung 13, welche auf Grund des elektrochirurgischen Eingriffs auf den Körper des Patienten erzeugt wird, genommen werden. Damit kann dafür Sorge getragen werden, dass auf Grund der Voraussetzungen bei dem Eingriff Eingriffslichterscheinungen 13 erzeugt werden, welche ein Analyseergebnis mit einer bestimmten Aussagekraft, insbesondere einer bestimmten Genauigkeit, ermöglichen.
Erfindungsgemäß wird eine Testeinrichtung TE zum Testen, insbesondere Kalibrieren, einer Analyseeinrichtung AE zur Analyse von Lichterscheinungen 13, 44 angegeben, welche durch ein HF-chirurgisches Instrument 11 hervorgerufen werden. Die Testeinrichtung TE weist ein Testobjekt 24 auf (dies kann in einfachen Ausführungsformen der Erfindung beinhalten, dass die Testeinrichtung von dem Testobjekt gebildet wird), wobei das Testobjekt zum Erzeugen einer Plasmatestlichterscheinung eingerichtet ist, deren Licht durch eine Lichtaufnahmeeinheit 14 aufnehmbar ist. In Ausführungsformen ist das Testobjekt 24 zum Erzeugen einer Plasmatestlichterscheinung 44 eingerichtet, indem mittels eines Instruments 11, welches in einem Zündabstand oder näher an dem Testobjekt 24 angeordnet wird, eine Plasmatestlichterscheinung 44 zwischen dem Testobjekt 24 und dem Instrument 11 erzeugbar ist. Das Testobjekt 24 ist bevorzugt dazu eingerichtet und bestimmt zur Anwendung bei der Vorbereitung und/oder der Durchführung einer geplanten Operation angeordnet und verwendet zu werden. Die Testeinrichtung TE ist bevorzugt eine Einrichtung eines HF-Chirurgiesystems S, welches ein HF-chirurgisches Instrument 11 und eine Analyseeinrichtung AE zur Analyse von Lichterscheinungen 13, 44 hervorgerufen durch das HF-chirurgisches Instrument 11 aufweist. Das Testobjekt 24 dient bevorzugt der Durchführung eines Funktionstests und/oder dem Ermitteln einer geeigneten Einstellung - vor und/oder während einer geplanten Operation - der Analyseeinrichtung AE und/oder anderer Einrichtungen des HF-Chirurgiesystems S durch Erzeugen einer Testlichterscheinung 44 an oder in dem Testobjekt 24, vorzugsweise an einer Testoberfläche 26 des Testobjekts 24. Damit kann überprüft werden, ob die Analyseeinrichtung AE bei der Operation einsatzfähig ist und/oder die Einsatzfähigkeit der Analyseeinrichtung AE kann damit verbessert werden. Es wird zudem ein Verfahren 100 zum Testen, insbesondere Kalibrieren, einer Analyseeinrichtung AE zur Analyse von Lichterscheinungen 13, 44 angegeben, welche Lichterscheinungen 13, 44 durch ein HF-chirurgisches Instrument 11 erzeugt werden. Das Verfahren 100 weist das Erzeugen einer Testlichterscheinung 44 zwischen einem Testobjekt 24 und einer Elektrode 10 auf. Das Licht der Testlichterscheinung 44 wird durch eine Lichtaufnahmeeinheit 14 aufgenommen. Das Verfahren 100 kann bevorzugt durch ein Mitglied des Operationsteams, insbesondere den Chirurgen oder einen Operationsassistenten ausgeführt werden.

### Bezugszeichenliste:

| | |
|---|---|
| 10 | Elektrode |
| 10a | Schaft |
| 10b | Kopf/Spitze |
| 10c | Isolation |
| 11 | Instrument |
| 11a | Handgriff |
| 12 | Gewebe/Körper des Patienten |
| 13 | Eingriffslichterscheinung/Plasma |
| 14 | Optische Faser/Lichtaufnahmeeinheit |
| 15 | Schlauch/Leitung |
| 16 | Lichtakzeptanzkegel |
| 17 | Lichteingang |
| 18 | Zündabstand |
| 19 | Adapter |
| 20 | Kanal |
| 21 | HF-Generator/Chirurgiegerät |
| 22 | Neutralelektrode |
| 23 | Analyseeinheit |
| 23a | Vorrichtung |
| 24 | Testobjekt |
| 25 | Gegenelektrode |
| 26 | Testoberfläche |
| 27 | Auswerteeinheit |
| 28 | Signalisierungseinheit |
| 29 | Steriler Bereich |
| 30 | Nicht steriler Bereich |
| 31 | Leitung |
| 32 | Leitung |
| 33 | Spülsystem |
| 34 | Fußschalter |
| 35 | Tasten |
| 36 | Abstandshalter |
| 37 | Träger |
| 38 | Öffnung |
| 39 | Raum |
| 40 | Wand |
| 41 | Auslassöffnung |
| 42 | Ausgang |
| 43 | Vorwiderstand |
| 44 | Testlichterscheinung |
| 45 | Spitze der Gegenelektrode |
| 46 | Rille |
| S | System |
| E | HF-Chirurgieeinrichtung |
| AE | Analyseeinrichtung |
| TE | Testeinrichtung |
| W | Winkel |
| A | Abstand |
| MA | Mindestabstand |
| L | Linie |
| H | HF-Strom |
| Li | Licht |
| Sg | Statussignal |
| M | Medium |
| Sc | Schutzgas |
| 100 | Verfahren |
| 101 | Schritt |
| 102 | Schritt |
| 103 | Schritt |

## Patentansprüche

1. Testeinrichtung (TE) zum Kalibrieren einer Analyseeinrichtung (AE) zur Analyse von Lichterscheinungen (13, 44) hervorgerufen durch ein HF-chirurgisches Instrument (11), wobei die Testeinrichtung (TE) ein Testobjekt (24) aufweist, wobei das Testobjekt (24) zum Erzeugen einer Plasmatestlichterscheinung eingerichtet ist, deren Licht durch eine Lichtaufnahmeeinheit (14) aufnehmbar ist, und wobei die Testeinrichtung (TE) dazu eingerichtet ist, aus bestimmten Lichtmerkmalen der Testlichterscheinung (44) eine Kalibrierung der Analyseeinrichtung (AE) zu bestimmen.

2. Testeinrichtung (TE) nach Anspruch 1, wobei das Testobjekt (24) eine Testoberfläche (26) zur Erzeugung der Testlichterscheinung (44) zwischen einer Elektrode (10), insbesondere einer Elektrode (10) des Instruments (11), und der Testoberfläche (26) aufweist.

3. Testeinrichtung (TE) nach einem der vorstehenden Ansprüche, wobei das Testobjekt (24) in einem sterilen Operationsbereich (29) anordenbar ist.

4. Testeinrichtung (TE) nach einem der vorstehenden Ansprüche mit einer Auswerteeinheit (27), welche dazu eingerichtet ist, anhand von Lichtmerkmalen des aufgenommenen Lichtes einer Testlichterscheinung (44), welche mittels des Testobjekts (24) erzeugt ist, zu bewerten, ob die Analyseeinrichtung (AE) funktionsfähig ist und/oder an den Anwender ein Signal oder einen Wert auszugeben, anhand dessen der Anwender bewerten kann, ob die Analyseeinrichtung (AE) funktionsfähig ist.

5. Testeinrichtung (TE) nach einem der vorstehenden Ansprüche, wobei die Testeinrichtung (TE) dazu eingerichtet ist, anhand der Testlichterscheinung (44) eine Empfindlichkeit der Analyseeinrichtung (AE) bei wenigstens einer Wellenlänge und/oder eine Transmissivität der Analyseeinrichtung (AE) bei wenigstens einer Wellenlänge zu ermitteln.

6. Testeinrichtung (TE) nach einem der vorstehenden Ansprüche, wobei die Testeinrichtung (TE) dazu eingerichtet ist, Signaturen aus Lichtmerkmalen des Lichts der Testlichterscheinung (44) zu ermitteln, die aus Intensitäten mehrerer Wellenlängen oder Teilspektren berechnet sind.

7. Testeinrichtung (TE) nach Anspruch 6, wobei jede Signatur aus Intensitätsverhältnissen ausgewählter für in den Plasmazustand übergegangene Teile des Materials der Testoberfläche (26) und/oder der Elektrode (10) und/oder der Atmosphäre zwischen der Elektrode (10) und der Testoberfläche (26) kennzeichnende Spektrallinien berechnet ist.

8. Testeinrichtung (TE) nach einem der vorstehenden Ansprüche, wobei das Testobjekt (24) wenigstens einen anhand dessen Emissionsspektrums ausgewählten Stoff enthält.

9. Testeinrichtung (TE) nach einem der vorstehenden Ansprüche, wobei das Testobjekt (24) wenigstens zwei unterschiedliche Testoberflächen (26) aufweist.

10. Testeinrichtung (TE) nach einem der vorstehenden Ansprüche, wobei das Testobjekt (24) gezielt Stoffe enthält, welche auch in dem zu untersuchenden oder zu behandelnden Gewebe (12) vorkommen oder vermutet werden, um eine Testlichterscheinung (44) zu erzeugen, aus welcher Lichtmerkmale bestimmt werden können, welche auch aus einer der Lichterscheinung (13) erzeugt durch Einwirkung des Instruments (11) auf das Gewebe (12) bestimmt werden können.

11. Testeinrichtung (TE) nach einem der vorstehenden Ansprüche, wobei die Testeinrichtung (TE) dazu eingerichtet ist, für wenigstens einen Parameter - beispielsweise einen Steuer- und/oder Auswerteparameter, beispielsweise zur Behandlung des Gewebes mit dem Instrument (11) - der Analyseeinrichtung (AE), der Testeinrichtung, des Testobjekts (24), des HF-chirurgischen Instruments (11), einer HF-Chirurgieeinrichtung und/oder einer sonstigen Einrichtung des Systems (S) für den HF-chirurgischen Eingriff, aufgrund des Tests mit der Testeinrichtung (TE) anhand der Testlichterscheinung (44) einen Wert oder einen Wertebereich vorzugeben.

12. Testeinrichtung (TE) nach einem der vorstehenden Ansprüche, wobei die Testeinrichtung (TE) dazu eingerichtet ist, eine Annäherung an oder eine Position der Elektrode (10) bei der Testoberfläche (26) zum Erzeugen einer Testlichterscheinung (44) automatisch zu erkennen und aufgrund dessen eine oder mehrere Einstellungen und/oder Einstellmöglichkeiten der Testeinrichtung (TE), der Analyseeinrichtung (AE), des Instruments (11), der HF-Chirurgieeinrichtung und/oder einer sonstigen Einrichtung für den HF-chirurgischen Eingriff, auszuwählen oder zu verändern.

13. Testeinrichtung (TE) nach einem der vorstehenden Ansprüche, wobei das Testobjekt (24) dazu eingerichtet ist, einen Mindestabstand (MA) zwischen einer Elektrode (10) und einer Testoberfläche (26) des Testobjekts (24) festzulegen und/oder wobei die Testeinrichtung (TE) dazu eingerichtet ist, die Atmosphäre zwischen einer Elektrode (10) und dem Testoberfläche (26) des Testobjekts (24) vorzugeben.

14. Testeinrichtung (TE) nach einem der vorstehenden Ansprüche, wobei das Testobjekt (24) dazu eingerichtet ist, eine Ausrichtung des Instruments (11), eines Adapters für das Instrument (11), einer Elektrode (10) und/oder einer Lichtaufnahmeeinheit relativ zu der Testoberfläche (26) festzulegen.

15. System mit einer Testeinrichtung (TE) nach einem der vorstehenden Ansprüche und einer Vorrichtung (19) zur Befestigung, durch den chirurgischen Anwender eines chirurgischen Instruments (11) oder seinen Assistenten, einer Lichtaufnahmeeinheit (14) der Analyseeinrichtung (AE) zur Lichtanalyse an dem Instrument (11) oder an einer Komponente (11a) des Instruments (11).

16. Nicht-chirurgisches Verfahren (100) zum Kalibrieren einer Analyseeinrichtung (AE), welche Analyseeinrichtung zur Analyse von Lichterscheinungen (13, 44), welche Lichterscheinungen (13, 44) durch ein HF-chirurgisches Instrument (11) bei der Einwirkung auf biologisches Gewebe (12) erzeugt werden, eingerichtet ist, wobei das Verfahren (100) das Erzeugen einer Testlichterscheinung (44) zwischen einem Testobjekt (24) und einer Elektrode (10) aufweist, deren Licht durch eine Lichtaufnahmeeinheit (14) aufgenommen wird, und wobei aus bestimmten Lichtmerkmalen der Testlichterscheinung (44) eine Kalibrierung der Analyseeinrichtung (AE) zu bestimmen.

## Claims

1. Test device (TE) for calibrating an analysis device (AE) for analysis of light appearances (13, 44) created by an RF surgical instrument (11), wherein the test device (TE) comprises a test object (24), wherein the test object (24) is configured for creation of a plasma test light appearance (44), the light of which can be received by a light receiving unit (14), and wherein the test device (TE) is configured to determine a calibration of the analysis device (AE) from specific light features of the test light appearance (44).

2. Test device (TE) according to claim 1, wherein the test object (24) comprises a test surface (26) for creation of the test light appearance (44) between an electrode (10), particularly an electrode (10) of the instrument (11) and the test surface (26).

3. Test device (TE) according to any of the preceding claims, wherein the test object (24) can be arranged in a sterile operation area (29).

4. Test device (TE) according to any of the preceding claims comprising an evaluation unit (27) configured to evaluate, based on light features of the received light of a test light appearance (44) created by means of the test object (24), whether the analysis device (AE) is operable and/or to output a signal or a value to the user based on which the user can evaluate whether the analysis device (AE) is operable.

5. Test device (TE) according to any of the preceding claims, wherein the test device (TE) is configured to determine a sensitivity of an analysis device (AE) at at least one wavelength and/or a transmissivity of the analysis device (AE) at at least one wavelength based on the test light appearance (44).

6. Test device (TE) according to any of the preceding claims, wherein the test device (TE) is configured to determine signatures from light features of the light of the test light appearance (44) that are calculated from intensities of multiple wavelengths or spectrum parts,

7. Test device (TE) according to claim 6, wherein each signature is calculated from intensity ratios of selected spectral lines being characteristic for parts of the material of the test surface (26) and/or electrode (10) and/or atmosphere between electrode (10) and test surface (26) transitioned into the plasma condition.

8. Test device (TE) according to any of the preceding claims, wherein the test object (24) comprises at least one substance selected based on its emission spectrum.

9. Test device (TE) according to any of the preceding claims, wherein the test object (24) comprises at least two different test surfaces (26).

10. Test device (TE) according to any of the preceding claims, wherein the test object (24) specifically comprises substances that are also present or that are expected to be present in the tissue (12) to be examined or to be treated in order to create a test light appearance (44) from which light features can be determined that can also be determined from a light appearance (13) created by the influence of the instrument (11) on the tissue (12).

11. Test device (TE) according to any of the preceding claims, wherein the test device (TE) is configured to define a value or a value range based on the test light appearance (44) due to the test with the test device (TE) for at least one parameter - for example a control and/or evaluation parameter, e.g. for treatment of tissue with the instrument (11) - of the analysis device (AE), the test device, the test object (24), the RF surgical instrument (11), an RF surgical device and/or any other device of the system (S) for the RF surgical intervention.

12. Test device (TE) according to any of the preceding claims, wherein the test device (TE) is configured to automatically determine an approach to or a position of the electrode (10) near the test surface (26) for creation of a test light appearance (44) and based thereon to select or modify one or more adjustments and/or adjustment possibilities of the test device (TE), the analysis device (AE), the instrument (11), the RF surgical device and/or any other device for the RF surgical intervention.

13. Test device (TE) according to any of the preceding claims, wherein the test object (24) is configured to define a minimum distance (MA) between the electrode (10) and a test surface (26) of the test object (24) and/or wherein the test device (TE) is configured to define the atmosphere between an electrode (10) and the test surface (26) of a test object (24).

14. Test device (TE) according to any of the preceding claims, wherein the test object (24) is configured to define an orientation of the instrument (11), an adapter for the instrument (11), an electrode (10) and/or a light receiving unit relative to the test surface (26).

15. System having a test device (TE) according to any of the preceding claims and a device (19) for attachment of a light receiving unit (14) of the analysis device (AE) for the light analysis to the instrument (11) or a component (11a) of the instrument (11) by the surgical user of a surgical instrument (11) or his/her assistant.

16. Non-surgical method (100) for calibrating an analysis device (AE), the analysis device is configured for analysis of light appearances (13, 44) created by the influence of an RF surgical instrument (11) on biological tissue (12), wherein the method (100) comprises the creation of a test light appearance (44) between a test object (24) and an electrode (10), the light of which is received by a light receiving unit (14), and wherein a calibration of the analysis device (AE) is determined from specific light features of the test light appearance (44) .

## Revendications

1. Dispositif de test (TE) destiné à l'étalonnage d'un dispositif d'analyse (AE) en vue de l'analyse d'apparitions de lumière (13, 44) provoquées par un instrument chirurgical HF (11), le dispositif de test (TE) présentant un objet de test (24), ledit objet de test (24) étant conçu pour générer une apparition de lumière de test plasma, dont la lumière peut être recueillie par une unité de réception de lumière (14), et le dispositif de test (TE) étant conçu pour déterminer un étalonnage du dispositif d'analyse (AE), à partir de caractéristiques de lumière précises de l'apparition de lumière de test (44).

2. Dispositif de test (TE) selon la revendication 1, dans lequel l'objet de test (24) présente une surface de test (26) destinée à générer l'apparition de lumière de test (44) entre une électrode (10), notamment une électrode (10) de l'instrument (11), et la surface de test (26).

3. Dispositif de test (TE) selon l'une des revendications précédentes, dans lequel l'objet de test (24) peut être installé dans une zone d'opération (29) stérile.

4. Dispositif de test (TE) selon l'une des revendications précédentes, comprenant une unité d'évaluation (27) qui est conçue pour évaluer, à l'aide de caractéristiques de lumière de la lumière reçue d'une apparition de lumière de test (44) générée au moyen de l'objet de test (24), si le dispositif d'analyse (AE) est opérationnel et/ou pour émettre un signal ou une valeur à destination de l'utilisateur, permettant à l'utilisateur d'évaluer si le dispositif d'analyse (AE) est opérationnel.

5. Dispositif de test (TE) selon l'une des revendications précédentes, dans lequel l'objet de test (24) est conçu pour déterminer, à l'aide de l'apparition de lumière de test (44), une sensibilité du dispositif d'analyse (AE) pour au moins une longueur d'onde et/ou une transmissivité du dispositif d'analyse (AE) pour au moins une longueur d'onde.

6. Dispositif de test (TE) selon l'une des revendications précédentes, dans lequel le dispositif de test (TE) est conçu pour déterminer des signatures à partir de caractéristiques de lumière de la lumière de l'apparition de lumière de test (44), qui sont calculées à partir d'intensités de plusieurs longueurs d'onde ou spectres partiels.

7. Dispositif de test (TE) selon la revendication 6, dans lequel chaque signature est calculée à partir de rapports d'intensité de raies spectrales sélectionnées, caractérisant des parties du matériau de la surface de test (26) et/ou de l'électrode (10) et/ou de l'atmosphère entre l'électrode (10) et la surface de test (26), qui sont passées à l'état de plasma.

8. Dispositif de test (TE) selon l'une des revendications précédentes, dans lequel l'objet de test (24) contient au moins une substance choisie sur la base de son spectre d'émission.

9. Dispositif de test (TE) selon l'une des revendications précédentes, dans lequel l'objet de test (24) présente au moins deux surfaces de test (26) différentes.

10. Dispositif de test (TE) selon l'une des revendications précédentes, dans lequel l'objet de test (24) contient de façon ciblée des substances qui sont également présentes ou dont on suppose la présence dans le tissu (12) à examiner ou à traiter, afin de générer une apparition de lumière de test (44), à partir de laquelle peuvent être déterminées des caractéristiques de lumière qui peuvent également être déterminées à partir d'une apparition de lumière (13) générée par l'action de l'instrument (11) sur le tissu (12).

11. Dispositif de test (TE) selon l'une des revendications précédentes, dans lequel l'objet de test (24) est conçu pour prédéfinir une valeur ou une plage de valeurs pour au moins un paramètre - par exemple un paramètre de commande et/ou d'évaluation, par exemple pour le traitement du tissu avec l'instrument (11) - du dispositif d'analyse (AE), du dispositif de test, de l'objet de test (24), de l'instrument chirurgical HF (11), d'un dispositif chirurgical HF et/ou d'un autre dispositif du système (S) destiné à l'intervention chirurgicale HF, sur la base du test avec le dispositif de test (TE) à l'aide de l'apparition de lumière de test (44).

12. Dispositif de test (TE) selon l'une des revendications précédentes, dans lequel le dispositif de test (TE) est conçu pour détecter automatiquement une approche de la surface de test (26) ou une position de l'électrode (10) près de la surface de test (26), afin de générer une apparition de lumière de test (44), et pour sélectionner ou modifier, sur cette base, un ou plusieurs réglages et/ou possibilités de réglage du dispositif de test (TE), du dispositif d'analyse (AE), de l'instrument (11), du dispositif chirurgical HF et/ou d'un autre dispositif destiné à l'intervention chirurgicale HF.

13. Dispositif de test (TE) selon l'une des revendications précédentes, dans lequel l'objet de test (24) est conçu pour définir une distance minimale (MA) entre une électrode (10) et une surface de test (26) du dispositif de test (TE), et/ou dans lequel le dispositif de test (TE) est conçu pour prédéfinir l'atmosphère entre une électrode (10) et la surface de test (26) de l'objet de test (24).

14. Dispositif de test (TE) selon l'une des revendications précédentes, dans lequel l'objet de test (24) est conçu pour définir une orientation de l'instrument (11), d'un adaptateur pour l'instrument (11), d'une électrode (10) et/ou d'une unité de réception de lumière, par rapport à la surface de test (26).

15. Système comprenant un dispositif de test (TE) selon l'une des revendications précédentes et un dispositif (19) destiné à la fixation, par l'utilisateur chirurgien d'un instrument (11) chirurgical ou par son assistant, d'une unité de réception de lumière (14) du dispositif d'analyse (AE), destiné à l'analyse de lumière, sur l'instrument (11) ou sur un composant (11a) de l'instrument (11).

16. Procédé non chirurgical (100) destiné à l'étalonnage d'un dispositif d'analyse (AE), lequel dispositif d'analyse (AE) est conçu pour l'analyse d'apparitions de lumière (13, 44), lesquelles apparitions de lumière (13, 44) sont générées par un instrument chirurgical HF (11) lors de l'action sur du tissu biologique (12), le procédé (100) présentant la génération d'une apparition de lumière de test (44) entre un objet de test (24) et une électrode (10) dont la lumière est reçue par une unité de réception de lumière (14), et selon lequel un étalonnage du dispositif d'analyse (AE) est défini à partir de caractéristiques de lumière précises de l'apparition de lumière de test (44).
